(19) Europäisches Patentamt
European Patent Office
Office européen des brevets

(11) **EP 2 310 829 B1**

(12) **EUROPEAN PATENT SPECIFICATION**

(45) Date of publication and mention of the grant of the patent:
**06.04.2016 Bulletin 2016/14**

(21) Application number: **09802318.7**

(22) Date of filing: **30.07.2009**

(51) Int Cl.:
*G06T 7/00* (2006.01)          *A61B 8/08* (2006.01)

(86) International application number:
**PCT/CA2009/001066**

(87) International publication number:
**WO 2010/012092 (04.02.2010 Gazette 2010/05)**

(54) **A SYSTEM AND METHOD FOR DETECTION, CHARACTERIZATION AND IMAGING OF HETEROGENEITY USING SHEAR WAVE INDUCED RESONANCE**

SYSTEM UND VERFAHREN ZUR ERKENNUNG, CHARAKTERISIERUNG UND ABBILDUNG VON HETEROGENITÄTEN MITHILFE EINER DURCH SCHERWELLEN HERVORGERUFENEN RESONANZ

SYSTEME ET PROCEDE DE DETECTION, DE CARACTERISATION ET D'IMAGERIE D'HETEROGENEITE PAR RESONANCE INDUITE PAR ONDES DE CISAILLEMENT

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **30.07.2008 US 129924 P**

(43) Date of publication of application:
**20.04.2011 Bulletin 2011/16**

(73) Proprietor: **Centre Hospitalier de l'Université de Montréal**
**Quebec H2W 1T8 (CA)**

(72) Inventors:
• **CLOUTIER, Guy**
**Repentigny**
**Quebec J5Z 4J6 (CA)**
• **SCHMITT, Cedric**
**Montreal**
**Quebec H2S 2S4 (CA)**
• **HADJ HENNI, Anis, Redha**
**Montreal**
**Quebec H2S 2V4 (CA)**
• **MONTAGNON, Emmanuel**
**Montreal**
**Quebec H2S 1E5 (CA)**

(74) Representative: **Ilgart, Jean-Christophe**
**BREVALEX**
**95, rue d'Amsterdam**
**75378 Paris Cedex 8 (FR)**

(56) References cited:
JP-A- 2000 187 024          US-A- 5 099 848
US-A- 5 592 085          US-A- 6 029 082
US-A- 6 037 774          US-A1- 2005 004 463

• **CHEN SHIGAO ET AL: "Quantifying elasticity and viscosity from measurement of shear wave speed dispersion", THE JOURNAL OF THE ACOUSTICAL SOCIETY OF AMERICA, AMERICAN INSTITUTE OF PHYSICS FOR THE ACOUSTICAL SOCIETY OF AMERICA, NEW YORK, NY, US, vol. 115, no. 6, 1 June 2004 (2004-06-01), pages 2781-2785, XP012072305, ISSN: 0001-4966, DOI: 10.1121/1.1739480**
• **PARKER KEVIN J ET AL: "A unified view of imaging the elastic properties of tissue", THE JOURNAL OF THE ACOUSTICAL SOCIETY OF AMERICA, AMERICAN INSTITUTE OF PHYSICS FOR THE ACOUSTICAL SOCIETY OF AMERICA, NEW YORK, NY, US, vol. 117, no. 5, 1 May 2005 (2005-05-01), pages 2705-2712, XP012072917, ISSN: 0001-4966, DOI: 10.1121/1.1880772**
• **SCHMITT C ET AL: "11C-5 Characterization of Time-Varying Mechanical Viscoelastic Parameters of Mimicking Deep Vein Thrombi with 2D Dynamic Elastography", ULTRASONICS SYMPOSIUM, 2007. IEEE, IEEE, PISCATAWAY, NJ, USA, 1 October 2007 (2007-10-01), pages 1009-1012, XP031195148, DOI: 10.1109/ULTSYM.2007.257 ISBN: 978-1-4244-1383-6**

- CHO SEUNG ET AL: "High-frequency torsional modal testing of a long cylinder by magnetostriction", APPLIED PHYSICS LETTERS, AIP, AMERICAN INSTITUTE OF PHYSICS, MELVILLE, NY, US, vol. 91, no. 7, 14 August 2007 (2007-08-14), pages 71908-71908, XP012100617, ISSN: 0003-6951, DOI: 10.1063/1.2769400
- HSIEH C P ET AL: "Novel technique of NDE in ceramic bearing balls", 19911208; 19911208 - 19911211, 8 December 1991 (1991-12-08), pages 891-894, XP010093787,
- KIRKPATRICK S J ET AL.: 'Imaging the Mechanical Stiffness of Skin Lesions by In Vivo Acousto Optical Elastography' OPTICS EXPRESS vol. 14, no. 21, 16 October 2006, pages 9770 - 9779, XP008142083

## Description

FIELD OF THE INVENTION

The present invention generally relates to dynamic elastography and material characterization using shear wave induced resonance.

BACKGROUND OF THE INVENTION

Dynamic elastography of tissues is a medical imaging technique which aims to identify and quantify the elasticity and viscosity of living tissues. Pathological structures, such as tumors (A. Samani et al., Phys. Med. Biol., 52, pp.1565-1576, 2007), blood clots (J. M. Rubin et al., Ultrasound Med. Biol., 25 (9), pp. 1179-1186, 2006) and other abnormal tissues having mechanical properties different from surrounding structures can be imaged and characterized by dynamic elastography.

Different methods for generating low frequency shear waves for dynamic elastography imaging of soft tissues exist. These methods employ external vibrating sources in contact with the structure to image (e.g., K. J. Parker et al., Ultrasound Med. Biol.,16, pp. 241-246,1990; R. Muthupillal et al., Science, 269 (5232), pp. 1854-1857. 1995), or an internal excitation using ultrasound radiation force to generate shear waves deeply into tissues (K. Nightingale et al., Ultrasound Med. Biol., 28, pp. 227-235, 2002; J. Bercoff et al, Appl. Phys. Lett., 84, pp. 2202-2204,2004). These techniques are used in sonoelasticity, magnetic resonance elastography, supersonic shear imaging and transient elastography to image, among other medical applications, heterogeneities such as tumors in tissues.

Document US 2005/004463 teaches the use of focused ultrasounds to induce vibrations in a medium. Shear elasticity and viscosity are calculated from the measured velocity of the material.

Document US 5 099 848 teaches a technique for breast imaging using vibration and ultrasound.

Document JP 2000/187024 teaches the use of waves to detect defects within a foam composite.

The article of S. Chen et al., "Quantifying elasticity and viscosity from measurement of shear wave speed dispersion", The journal of the acoustical society of America, 115 (6), p. 2781-5, 2004 discloses the use of focused ultrasounds to produce a radiation force that generates a shear waves within a bulk medium. The speed of propagation and the amplitude of the shear wave are used to characterize the bulk medium.

The article of K. J. Parker et al., "A unified view of imaging the elastic properties of tissue", The journal of the acoustical society of America, 117(5), p. 2705-12, 2005 discloses the use of propagative shear waves into a bulk medium in order to observe and measure the resonance of this medium. The presence of a mechanical inhomogeneity within another medium will change the pattern of the eigenmodes in the first medium. This change in the overall pattern is imaged to observe the presence or the absence of a mechanical heterogeneity.

The article of C. Schmitt et al., "Characterization of Time-Varying Mechanical Viscoelastic Parameters of Mimicking Deep Vein Thrombi with 2D Dynamic Elastography", Ultrasonics Symposium, p. 1009-12, 2007 discloses the tracking of the propagation of a harmonic plane shear wave.

Documents US 5 592 085 and US 6 037 774 teach the use of shear wave to induce alterations in the phase of an NMR signal to enhance imaging.

However, in the case of the ultrasound radiation force generation technique, the method of inducing vibrations can result in a local temperature increase in the tissue to which the vibrations are being applied. Moreover, in known dynamic elastography methods, there is some reliance on imaging techniques such as ultrasound to image the tissue or the heterogeneity being vibrated. Therefore, if the contrast in the image or the mechanical properties between the heterogeneity and the surrounding tissue is not adequate, the heterogeneity cannot be clearly detected.

Whatever the method of imaging implemented, it is found that the quality of the mechanical excitation of the medium affects the quality of the elastographic image obtained. Indeed, the signal-to-noise ratio of measured displacements following mechanical excitation and the aptitude of the shear wave to be propagated deeply into the medium are examples of factors impairing image quality. To counterbalance these effects, the various techniques of shear wave generation in soft tissues (e.g., generation by impact, with contact and by radiation force) need to be optimized for imaging certain confined pathologies such as thromboses, aneurysms and tumors.

Therefore, it is desired to overcome or reduce at least some of the above-described problems.

SUMMARY OF THE INVENTION

The invention is defined in the independent claims 1 and 9.

Preferred embodiments are defined the dependent claims.

There is provided a system for inducing resonance of a first medium within a second medium, the first and second medium having different mechanical properties, the system comprising: a shear wave generator; and a detection unit; wherein the shear wave generator generates shear waves, the shear waves are applied to the second medium and are oriented with respect to the first medium according to a geometry of the first medium, the shear waves selectively inducing mechanical resonances of the first medium, the detection unit detecting the induced mechanical resonances.

There is further provided a system comprising: a vibration source, a container for a sample; the container being connected to the vibration source, a vibration sensor; and a processor, wherein the vibration source generates shear waves that induce vibrations and the resonance of the sample in the container, the vibration sensor measuring the sample vibrations and resonances, the processor determining viscoelasticity of the sample from the resonances.

There is further provided a method for imaging a heterogeneity within a body, comprising selectively inducing resonance of the heterogeneity by applying selected shear waves to the body; measuring the resonance of the heterogeneity to obtain displacement spectra; and deriving an image of the heterogeneity from eigenmodes of the displacement spectra.

There is further provided a method for measuring the viscoelastic properties of a heterogeneity within a body, comprising inducing resonance of the heterogeneity by applying shear waves to the body, measuring the resonance of the heterogeneity to obtain at least one of: displacement, velocity and acceleration data; and deriving the viscoelastic properties of the heterogeneity from the measured data.

There is further provided a method of dynamic elastography comprising inducing resonance of a body by applying selected shear waves on the body.

There is further provided a system of dynamic elastography of a tissue, comprising a shear wave generator; a detection unit; and a processor; wherein the shear wave generator generates shear waves, the shear waves are applied to the tissue, the shear waves selectively inducing mechanical resonances of the tissue, the detection unit detecting the induced mechanical resonances; the processor obtaining elasticity and viscosity of the tissue from the resonances.

Other objects, advantages and features of the present invention will become more apparent upon reading of the following non-restrictive description of specific embodiments thereof, given by way of example only with reference to the accompanying drawings.

BRIEF DESCRIPTION OF THE DRAWINGS

Further aspects and advantages of the present invention will become better understood with reference to the description in association with the following in which:

Figure 1 is a block diagram of a system according to a first embodiment of the present invention;

Figure 2 is a block diagram of a system according to a second embodiment of the present invention;

Figure 3 illustrates a shear wave generation system used in the system of Figures 1 or 2 according to an embodiment of the present invention;

Figure 4a illustrates a shear wave generation system used in the system of Figures 1 or 2 according to another embodiment of the present invention. Figure 4b is a schematic illustration of the shear wave generated in Figure 4a. Figure 4c illustrates details of the shear wave generation system of Figure 4a;

Figures 5a - 5c illustrate generation of non-focal torsional (circumferential) shear waves according to an embodiment of the present invention;

Figure 6 illustrates generation of focalized torsional (circumferential) shear wave according to an embodiment of the present invention;

Figures 7a and 7b illustrate a two-dimensional generation of a focalized shear wave according to an embodiment of the present invention;

Figure 8a illustrates a device using the two-dimensional system of Figures 7a and 7b to analyze human breasts. Figure 8b shows a detail of Figure 8a. Figure 8c is a plan view of Figure 8b; Figure 8d shows (left) B-mode image of a breast with a suspected benign solid lesion (white arrow), (right) an image obtained using the system of Figure 4c;

Figure 9 is a flowchart of a method of imaging an heterogeneity using shear wave induced resonance according to an embodiment of a second aspect of the present invention;

Figure 10 illustrates an alternative embodiment of the system of Figures 1 and 2;

Figure 11a illustrates a skin tumor (taken from S. J. Kirkpatrick et al., "Imaging the mechanical stiffness of skin lesions by in vivo acousto-optical elastography", Optics Express, 14 (21), pp. 9770-9779, 2006). Figure 11b illustrates measurement of the skin tumor of Figure 11a according to an embodiment of the present invention;

Figure 12 illustrates scattering of a plane shear-horizontal wave by a cylinder;

Figure 13 illustrates (a) measured, and (b) simulated displacement spectra, obtained at two different positions within a resonant inclusion contained in a sample;

Figure 14 illustrates (left) measured, and (right) simulated eigenmodes (stationary normalized displacement fields) corresponding to three identified eigenfrequencies of Figure 13;

Figure 15 is an ultrasound B-mode intensity echographic image of the inclusion which resonances are shown in Figure 14;

Figure 16 illustrates (left) three-dimensional representations of the measured displacement eigenmodes, and (right) displacement stationary profiles measured along the X axis crossing the circular cross-section of the inclusion;

Figure 17 illustrates (a) the first resonance frequency, and (b) the Q-factor (quality factor) evolutions with respect to the elasticity and viscosity of the cylindrical inclusion of Example 2, (c) illustrates a flow chart of a method used in Example 2 for determining the viscoelasticity of a heterogeneity (inclusion) or that of a homogeneous material;

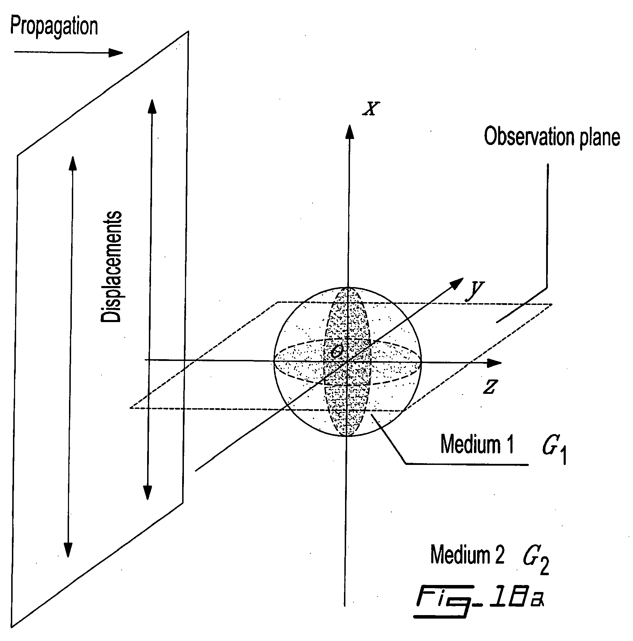

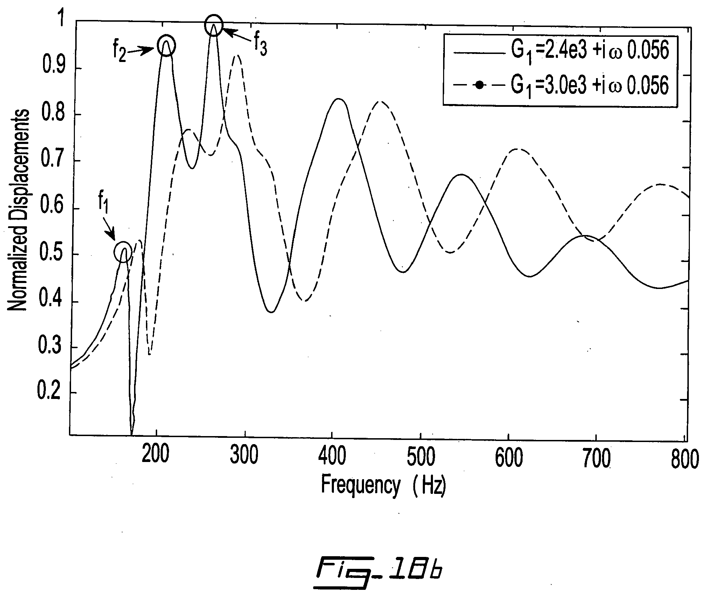

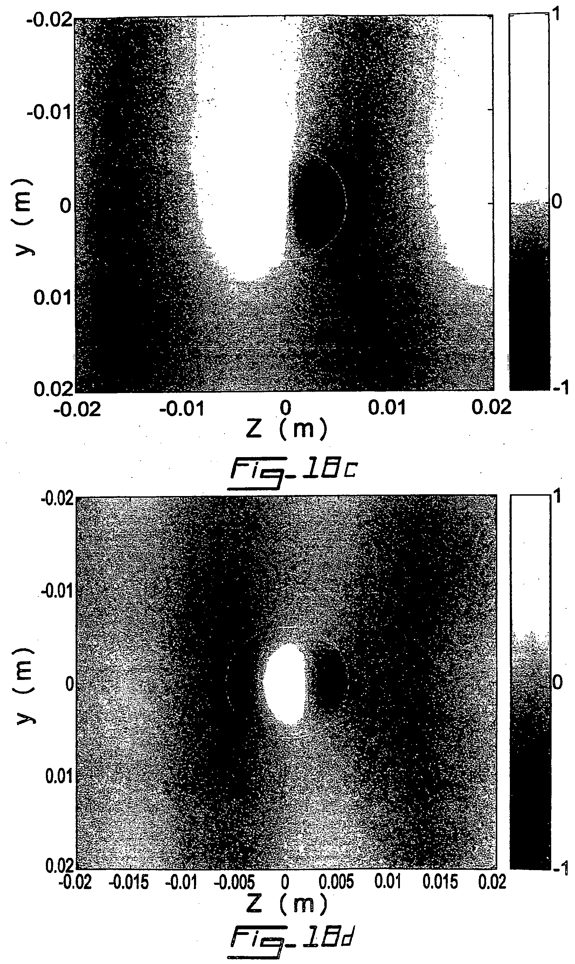

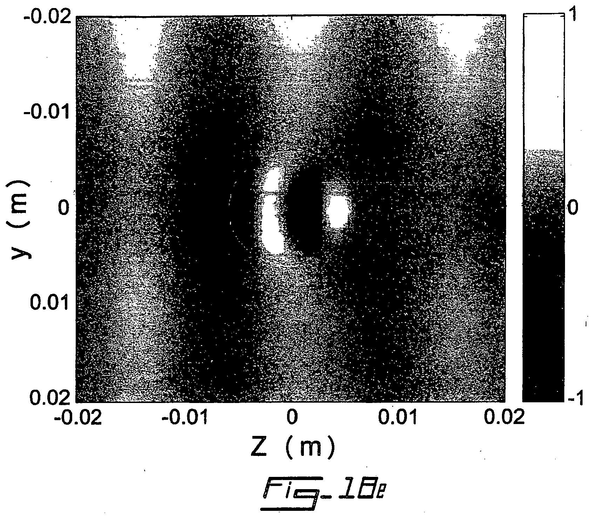

Figures 18 illustrate a) the geometrical configuration for the scattering of a plane shear wave by a spherical inclusion; b) the spectrum of displacements computed at $(0,1mm,1mm)$, inside the sphere, from 100Hz to 800 Hz for two different shear moduli, $G_1$ = 2.4 k$Pa$ (black) and $G_1$ = 3.0 k$Pa$ (red). In the first case, discrete resonance frequencies are observed at: $f_1$ = 146.1 Hz, $f_2$ = 178.6 Hz, et $f_3$ = 227.3 Hz ; c) the normalized displacement map at $f_1$ =160.3$Hz$ in the plane (0, $y$, $z$); d) the normalized displacement map at $f_2$= 205.4 $Hz$ in the plane (0, $y$, $z$), and e) the normalized displacement map at $f_3$ = 259.2Hz in the plane (0, $y$, $z$).

Figures 19 illustrate a) the geometrical configuration for an elliptic cylindrical inclusion; b) the spectrum of displacements computed at (0,1m$m$ , 0.1 m$m$) inside the inclusion, from 100Hz to 400 Hz for two different shear modulus $G_1$ =1.2 k$Pa$ (red) and $G_1$ = 2.4 k$Pa$ (black). In the first case, discrete resonance frequencies are: $f_1$ =146.1$Hz$, $f_2$ = 178.6$Hz$, $f_3$ = 227.3$Hz$., c) the normalized displacement map at $f_1$ =146.1$Hz$, d) the normalized displacement map at $f_3$ =178.6$Hz$; and e) the normalized displacement map at $f_3$= 227.3$Hz$.

Figures 20 illustrates (a) displacement spectrum of an elliptical inclusion with major axis a = 10.5 mm, and minor axis b = 8 mm; (b) displacement field at the first resonance frequency of 61 Hz, and (c) second eigenmode of vibration at 86 Hz (viscoelastic parameters were: $\mu_1$= 2500+$i\omega$0.056 for the inclusion and $\mu_2$ =17000+$i\omega$0.07 for the surrounding medium), in Example 3;

Figure 21 illustrates a sphere geometry used as an inclusion in Example 4 where the inclusion is submitted to a torsional shear wave to induce circumferential resonance;

Figures 22 illustrate measured stationary displacement fields obtained in Example 4 at (a) a first resonance mode at 130 Hz, and (b) a second resonance mode at 169 Hz;

Figures 23a-23d illustrate experimentally measured focalized shear waves into a homogeneous phantom using a hollow spherical cap of Example 5;

Figures 24a-24c illustrate simulation results of first resonance eigenmodes of the spherical geometry of Figure 21, corresponding to the first, second and third eigenfrequency at (a) 140.6 Hz, (b) 183.0 Hz, and (c) 225.0 Hz; and

Figures 25 illustrates three-dimensional views of (a) the first eigenmode obtained at 120 Hz, (b) the second eigenmode at 176 Hz, (c) the third eigenmode at 261 Hz, of a spherical heterogeneity embedded into a phantom vibrating under the action of focalized shear waves.

DETAILED DESCRIPTION OF THE INVENTION

This invention is not limited in its application to the details of construction and the arrangement of components set forth in the following description or illustrated in the drawings. The invention is capable of other embodiments and of being practiced or of being carried out in various ways. Also, the phraseology and terminology used herein is for the purpose of description and should not be regarded as limiting. The use of "including", "comprising", or "having", "containing", "involving" and variations thereof herein, is meant to encompass the items listed thereafter as well as additional items.

In broad terms, there is provided a method and a system for inducing resonance of a heterogeneity, i.e. for example an ab-normal tissue, vein thrombosis, a blood clot, a tumor and an inclusion within a body for the detection and monitoring of the induced resonance. The heterogeneity may be partially or completely included within the body. The mechanical resonance of the heterogeneity is induced by shear waves oriented with respect to the heterogeneity in such a way as to induce resonance of the heterogeneity.

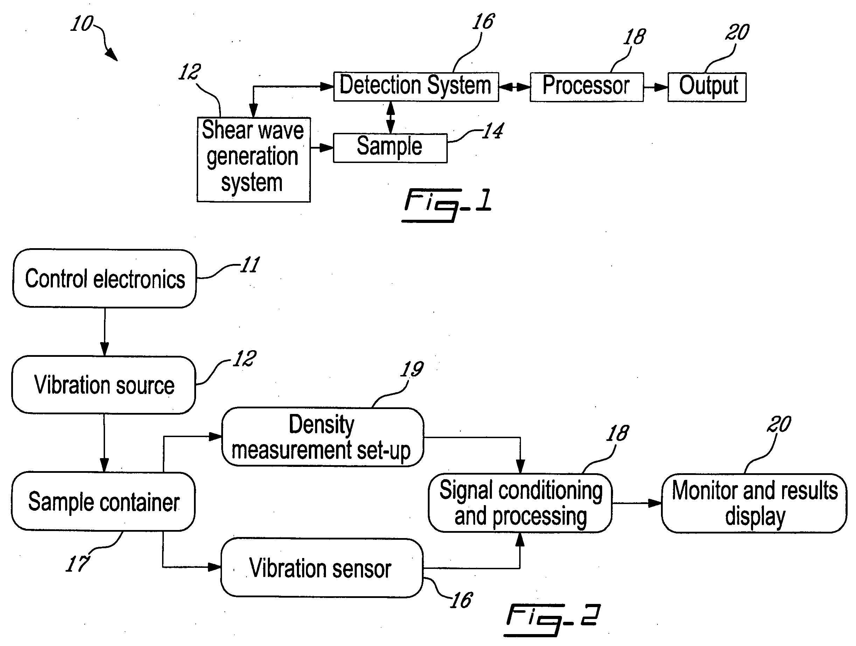

Referring initially to Figure 1, a system 10 generally comprises a shear wave generation unit 12 for generating shear waves to be applied to a sample 14 including a heterogeneity incorporated therein or thereon, and a detection unit 16 for detecting the resonance of the heterogeneity. A processor 18 can be used to process the detected resonance data, and an output for outputting the processed detected resonance, e.g. as an image, a data or alerts for example.

The shear wave generation unit 12 includes a shear wave generator having a contact surface which is made to move in such a way as to generate shear waves.

In an embodiment of the present invention, the shear wave generator is selected so that shear waves propagating into the sample 14 induce resonances in the heterogeneity, according to the geometry of the heterogeneity: in the case of an elongated inclusion having a longitudinal axis, shear waves generated along a plane substantially parallel to the longitudinal axis of the heterogeneity induce, at certain frequencies, resonance in the heterogeneity only, and, in the case of a spherical heterogeneity, torsional shear waves, i.e. with circumferential wave polarization, are used to induce, at certain eigenfrequencies, the torsional resonance of spherical (or spheroid) heterogeneity.

The output device 20 may include a display for displaying the output data such as the displacement spectra, the measured resonance frequencies, the eigenmode images, i.e. the stationary displacement images obtained when the sample is excited at each resonance frequency, the low frequency vibration signal measured by an accelerometer, the extracted inclusion geometry, and the estimated viscoelasticity of the sample media, i.e. of the resonant inclusion and its surrounding medium.

The system 10 may be contained within a single housing to form a single apparatus or may comprise a device comprising separate parts. Single housing embodiments may be suitable for bedside or self-monitoring applications, for example.

A block diagram of an embodiment of the system suitable for rheological and mechanical characterization when sampling is preferable, for example, in the study of blood coagulation or characterization of industrial material properties is illustrated in Figure 2. It comprises a vibration source 12, to induce the shear waves and thus the vibrations and the resonance of the sample to be characterized, and a sample container 17 in which the tested material, in a solid or semi-solid state, is placed. The sample is geometrically conformed to the container shape which is arbitrary. The container 17 is connected to the vibration source 12 to force the sample vibration and resonance. A vibration sensor 16 is used to measure, in contact or without contact, the sample vibrations and resonances in displacement, velocity or acceleration modes. The sensor 16 can be an ultrasound probe, a microphone, a laser beam or any other technology. A weight and volume sensor 19 is used to measure the sample density. An electronic control unit 11 is used to induce and measure vibrations and resonances. Such a system can characterize soft and viscous materials currently met in industries like food, pharmacy, polymers, chemistry, hydrocarbons... Viscoelasticity determination is based on the resonance spectrum properties of the sample.

Alternatively, the system can be joined with or incorporated within existing elastography imaging systems using ultrasound (e.g., an ultrasound scanner with a dynamic elastography or sonoelastography imaging module) or within an existing magnetic resonance imaging system. This may be particularly suitable for medical applications.

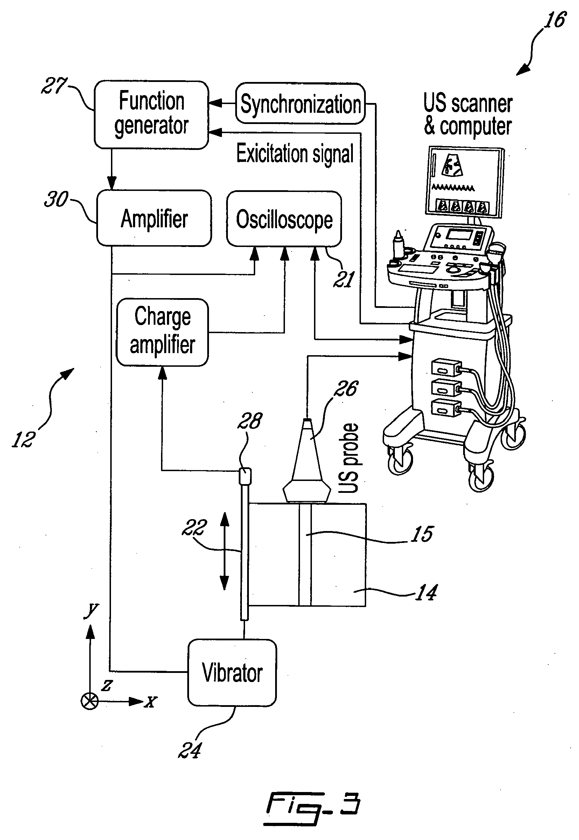

The system allows heterogeneities to be accurately imaged and their material properties accurately characterized. The system may further have a large number of applications such as in medical imaging, medical diagnosis and medical therapeutic as well as in the characterization of material rheological properties. In an embodiment illustrated in Figure 3, the shear wave generation unit 12 includes a rigid plate 22 having a surface for contacting the sample 14, the plate 22 being connected to a vibrator 24 (e.g. Brüel&Kjær, type 4810, Nærum, Denmark). Movement of the vibrator 22 and hence the plate 22 is powered by a function generator 27 (Agilent, model 33250A, Palo Alto, CA, USA), or any other suitable device, and measured by an accelerometer 28 (Brüel&Kjær, type 4375, Naerum, Denmark). An amplifier 30 (e.g., standard 2706 from Brüel&Kjær, Nærum, Denmark) is used to amplify the signal being supplied to the vibrator 24 from the function generator 26. In use, the vibrator 24 induces actuation of the plate 22 which generates shear waves which propagate into the sample 14 and interact with the inclusion 15 to induce its resonance. The detection unit 16 is an ultrasound imaging system and is arranged to detect a backscattered ultrasound signal from the sample 14 via an ultrasound probe 26.

The sample 14 comprises a first mass of material having a second mass 15 of material partially or completely included therein, the first and second masses of material having different mechanical properties. This embodiment of the present system is well-suited, although not limited, to biomedical applications such as the imaging of deep vein thromboses within the body or other inclusions or heterogeneities. For example, the sample can be the leg of a patient with partially or completely embedded cylindrical or ellipsoid blood clots. In this case, the ultrasound probe 26 can be positioned on the skin of the leg of the patient.

In this embodiment, the heterogeneity 15 is a cylindrical inclusion embedded in the sample 14, and the contact surface of the plate 22 is positioned approximately parallel to the longitudinal axis of the inclusion 15 in order to generate shear waves propagating substantially perpendicularly to the longitudinal axis and polarized parallel to the inclusion axis: these are shear-horizontal (SH) waves.

When using an ultrasound imaging system 16, the direction of the applied ultrasound signal is preferably parallel to the shear wave polarization. The resultant displacement spectra of the inclusion 15 are obtained via the probe 26 and processed by the processor 18 in order to obtain the heterogeneity resonance frequencies and the images of the heterogeneity eigenmodes, as will be described in further detail below.

Further processing of the signals acquired by the ultrasound probe 26 is either performed by the processor 18, or another processor such as a computer or any other digital signal processor. The further processing comprises processing the detected signals using a cross-correlation algorithm or a Doppler based algorithm for example applied to the acquired RF signals to obtain the displacement field and its temporal evolution. The processor 18 may also include a memory for storing the digital signal, storing instructions for the data processing, and storing output data of the data processing. The processor 18 may also include an oscilloscope, incorporated as an electronic card, to process the acquired low frequency excitation signal. Alternatively, the oscilloscope 21 may be separated from the processor 18 and may be used for digitizing a signal received from an accelerometer, which data is then processed by the processor 18, as shown in Figure 3 for example.

It will be clear to skilled persons that other types and configurations of the shear wave generator and its contact surface are possible. For example, instead of a rigid plate, a cylindrical plate, a portion of a cylinder, a spherical cap, an elongate member such as a one-dimensional wire or any other type of shape or configuration of the contact surface may be used. It is also possible to generate suitable shear waves deeply into the sample using an adaptive ultrasound radiation force technique.

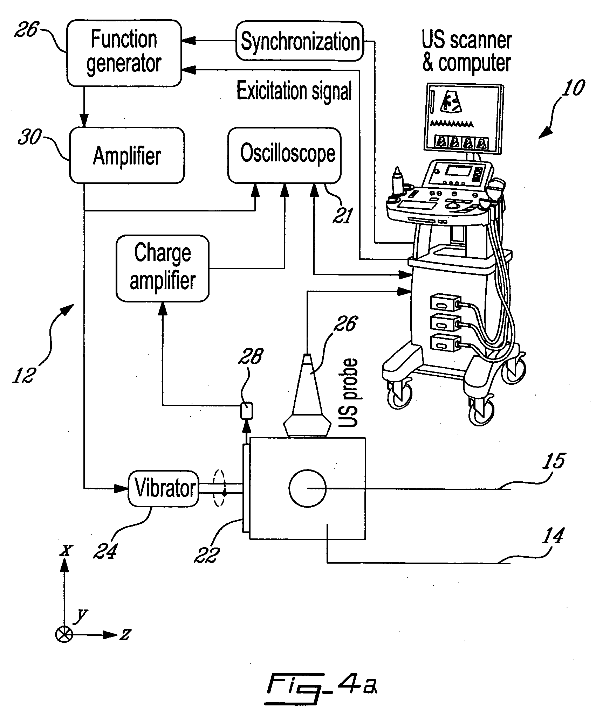

Besides ultrasound based units as used in Figure 3 (or in Figure 4a discussed below), alternative detection units 16 may be used for the detection and/or measurement of the displacement of the sample 14 including the heterogeneity 15. Such units include those based on magnetic resonance imaging, for example. For both cylindrical or spherical-shaped inclusions, a magnetic resonance imaging system can be used to measure and image the three-dimensional displacement maps into the sample. Alternatively, systems which measure the displacements at the surface or near the surface of the sample are also included within the scope of the present invention, such as laser beams, acousto-optical systems, high sensitivity microphones, cameras, air coupled ultrasound, velocity or acceleration sensors, etc.

Alternatively, the system 10 may include one or several separate transducers/receivers working as a low frequency shear wave generator and as a receiver.

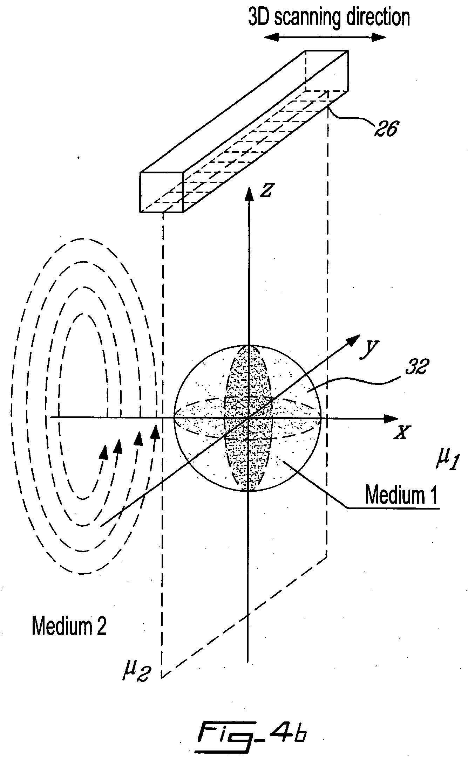

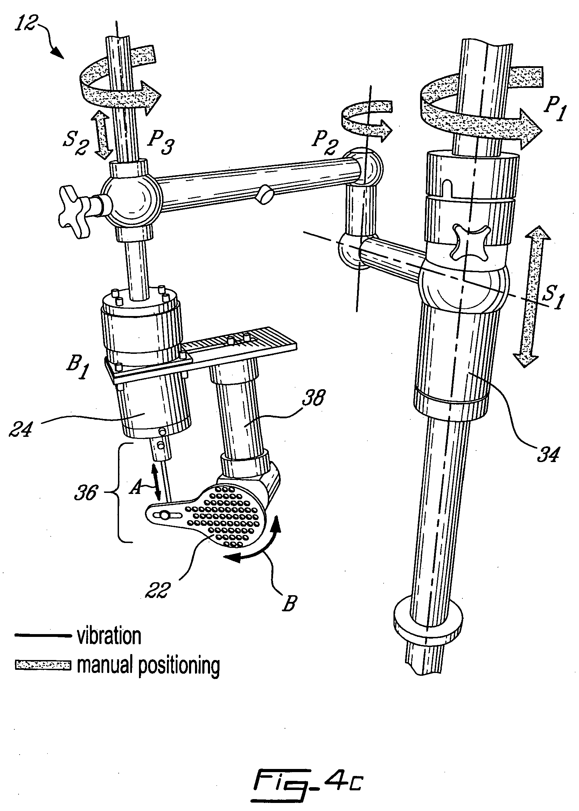

Figures 4a-4c illustrate another embodiment of a shear wave generation unit, in the case of spherical or nearly spherical inclusions or heterogeneities 15 (shown as Medium 1 in Figure 4b) in a body (shown as Medium 2 in Figure 4b). In this embodiment, the rigid plate 22 is arranged to rotate, rather than reciprocate backwards and forwards, in order to produce torsional shear waves polarized in the circumferential direction. In this spherical inclusion configuration, the generated displacement is circumferential and the shear waves propagate following an axis X crossing the inclusion (see Figure 4b). If an ultrasound probe 26 is used to measure and image displacements, the ultrasonic beams are preferably perpendicular to the wave propagation axis, as shown in Figure 4b. Figure 4c illustrates details of the shear wave generator of Figure 3. As shown, the rigid plate 22 is positionable near the sample, by handle 38, using a rigid stable arm 34 with three pivot connections (P1, P2 and P3), two sliding connections (S1 and S2) and one ball joint (B1). The pivot connections (P1, P2 and P3) and the sliding connections (S1 and S2) allow a manual positioning. The vibration is generated in translation by the linear vibrator 24 (see arrow A) and converted to a rotational motion (see arrow B) by the use of an off-center connection 36.

Further embodiments of the shear wave generation system 12 are possible. For example, different configurations of the shear wave generator can be employed to generate torsional (rotational) shear waves, into the sample.

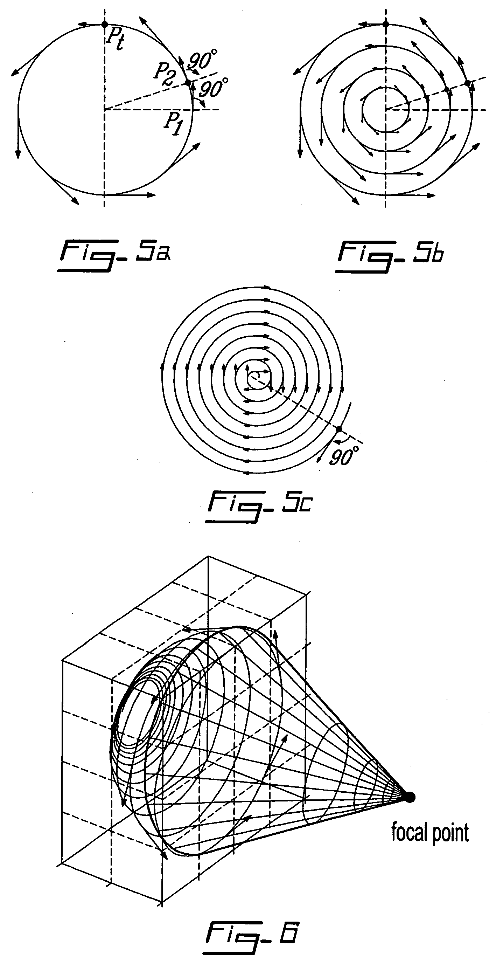

Figures 5a, 5b and 5c illustrate the generation of different non-focalized shear wave. A shear wave source without focalization can be induced by a local mechanical motion relative to the sample in a direction tangential to a circle or ring (Figure 5a), concentric circles (Figure 5b) or a spiral (Figure 5c). At each point ($P_1$, $P_2$, ..., $P_t$), illustrated in Figure 5a, and localized in a 2D plane according to a known particular shape, the superposition of these local motion sources induces a global rotational shear around the circle (or spiral) centre. In practice and by using an external device, the non-focalized shear wave pattern of Figure 5a can be obtained by rotational vibration of a rigid circle wire or a ring in contact with the sample for example, whereas the second shear wave pattern of Figure 5b can be generated with a rigid plate subjected to rotational vibration for example. In the third shear wave pattern of Figure 5c, a spirally shaped rigid membrane rotates around its fixed axis due to a vibration applied to its free extremity. All illustrated configurations can also be generated by using an internal source, such as radiation pressure for example, by applying the radiation at different locations described by a known shape.

In another embodiment, illustrated in Figure 6, 3D torsional shear waves focalised at a focal point or in a focal region are generated using an external device such as a hollow spherical cap for example. Submitted to rotational vibration, the inner surface of this spherical cap volume generates concentric shear wave lines which, due to sphere concavity, focus at a particular depth. The same pattern can be obtained with the radiation force technique by monitoring, for example, the wave generation of a circular multi-array ultrasound probe. Alternatively, a line excitation source, i.e. a rigid and annular vibrating source in contact with the body, could be used to induce the focalization of the waves.

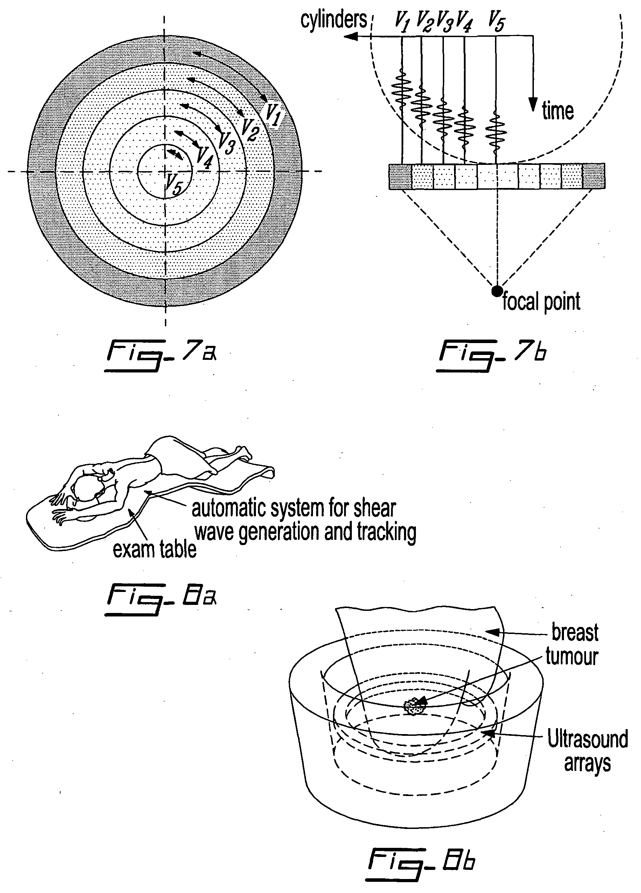

Another source of shear wave generation is illustrated in Figures 7a and 7b. This shear wave generation technique differs from that of Figure 6 in that an appropriate time-delay law is introduced to the vibration of concentric rings to force the shear wave to focalize at a target depth. As a result, a local motion source of the tissue is generated on a 2D plane contrary to a 3D surface as in Figure 6. This type of shear waves (Figures 7) can be generated either from the surface of the sample by vibration of concentric rings positioned adjacent and in contact with the sample or in-depth with radiation force focalization.

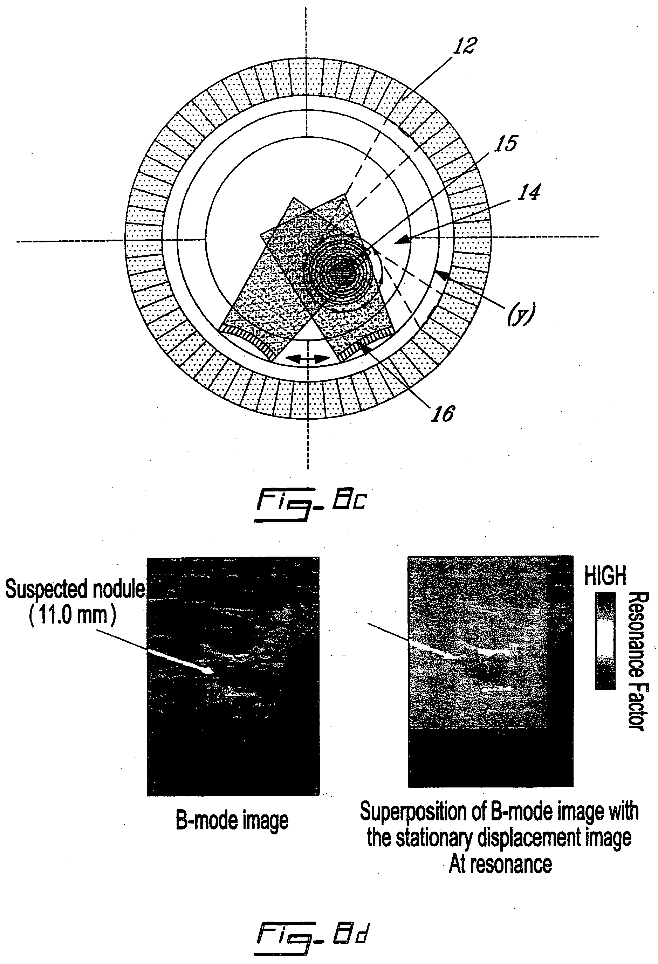

One embodiment of an in vivo automatic shear wave generation system with radiation force focalization for human breast investigations is illustrated in Figures 8a, 8b, 8c and 8b. In this example, the shear wave generation system is housed within an ergonomic examination table adapted to support a reclining patient and having an opening or openings for receiving the patient's breast or breasts or any other portion of the patient's anatomy under observation (see Figure 8a). Within each opening is mounted a moveable (in translation and rotation) dedicated circular ultrasound array and a curved/circular array connected to an ultrafast ultrasound scanner, as shown in Figures 8b and 8c, which in use surround the patient's part of the body under observation. Figure 8c illustrates a circular array for circular shear wave generation 12 (shear waves are generated following a closed path, i.e. circular or more complex shapes, surrounding the heterogeneity to induce shear waves focalization) and a curved array for RF acquisition 16 in plane view. This curved array 16 can be moved following a positioning axis (Y) around the patient's breast 14. Suitable shear waves for inducing resonance can be generated by ultrasound radiation force using an adapted beam-forming technique and by applying a suitable time-delay law to ultrasound elements, for example, to generate torsion following a circular path around a substantially spheroid pathology. Figures 8d shows in vivo images of a benign breast lesion (white arrow). The image on the right hand side was obtained using torsional waves generated by the system of Figure 4c.

It will be appreciated that although the shear wave focalization described above has been described in relation to inducing resonance in confined heterogeneities, it can also be used to perform dynamic elastography or sonoelastography of homogeneous materials such as the liver or any other organs. In this case, the focalization allows the enhancement of the elastographic images and improvement of the mechanical characterization, since the energy is focalized and tissue displacements optimized.

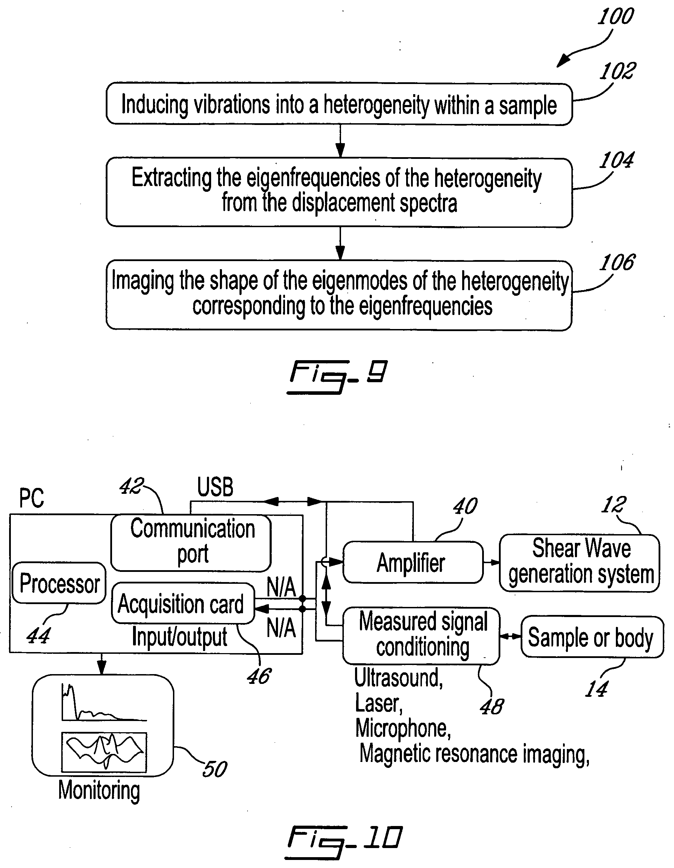

The present invention also extends to a method 100 of imaging inclusions within a material. As illustrated in Figure 9, a method 100 according to an embodiment of a second aspect of the present invention broadly comprises the steps of i) inducing vibrations into a heterogeneity within a sample (step 102), ii) extracting the resonance frequencies of the heterogeneity (the eigenfrequencies) from obtained displacement spectra (step 104), and iii) imaging the shape of the heterogeneity eigenmodes (i.e., the stationary displacement field obtained when the incident wave has an eigenfrequency as central frequency) (step 106).

In step 102, vibration of the heterogeneity is forced by the scattering of properly selected shear waves in harmonic or transient regime. This is achieved by applying a shear wave to the sample containing the inclusion or directly to the inclusion in case when, for example, the inclusion is not completely embedded within the sample. In the case of elongated inclusions, this may be achieved by propagating a shear wave polarized following the longitudinal axis and travelling in a direction substantially transverse to the longitudinal axis of the inclusion, and the maximum displacement is measured along the longitudinal inclusion axis. For substantially spherical inclusions, this may be achieved by the interaction of a torsional shear wave with the inclusion.

Typically, for biological applications, the frequency of the shear wave ranges between a few hertz and 3.5 kHz depending on the size and material properties of the inclusion, for example between 50 and 1000 Hz. For the measurement of viscoelasticity of a sample within a container body, the frequency range is typically between 0.01 Hz and 10 kHz depending on the precision of vibrations measurement technique, for example between 10 Hz and 2000 Hz.

In biological applications, heterogeneities of a size between 1 mm and 150 mm in diameter. For example between 5 mm and 20 mm, are detectable by the present system and method. In industrial applications for viscoelasticity measurements for example, samples of a diameter between 1 mm and 1000 mm, for example between 5 mm and 30 mm, may be assessed by the present system and method.

In step 104, displacement spectra of the inclusion are obtained by processing the detected ultrasound signals (if an ultrasound scanner is used) or any other signal resulting from a measurement device (like microphone, laser, air-coupled ultrasound etc). In the case of ultrasound detection, this is achieved by calculating the displacements using, for example, a normalized cross-correlation algorithm applied to the detected ultrasound radio frequency (RF) signals in a manner known in the art. The eigenfrequencies are identified by applying a Fourier transform to the displacement temporal data in order to extract the frequencies for which displacements are maximum. These eigenfrequencies are stored for step 106. Optionally, the displacements may be filtered before treatment for more precision. The eigenfrequency values and the shape of the displacement spectra, determined by calculating the quality factor or Q-factor of the spectrum, are used to characterize the viscoelasticity of the inclusion. This is achieved by solving, for example, an inverse problem involving the measured data and a theoretical model, as will be described hereinbelow in relation to Example 2 for a cylindrical heterogeneity.

In step 106, the shape of the eigenmodes are imaged by exciting the inclusion at each of the identified eigenfrequencies. This can be done by selectively choosing the appropriate shear-wave frequency or by using a chirp excitation (continuous change in frequency as a function of time). The temporal displacement field (obtained by the interaction of the incident shear wave with the inclusion) is measured and Fourier transformed to get the stationary displacement images of the eigenmodes. These images can be used to visually or numerically segment the inclusion despite the absence of magnetic or echogenicity contrast between the heterogeneity and the surrounding medium. Indeed, since resonance is confined into the inclusion, it is possible to perfectly distinguish the inclusion boundary movements and discriminate the heterogeneity from its surrounding medium.

For each measured eigenfrequency, the shear wave source thus generates a harmonic (or pseudo-harmonic, chirp, etc) or a transient wave with a central frequency equal to the identified eigenfrequency. The imaging system then measures, for each frequency, the displacement field evolution over time. For medical applications and if an ultrasound imaging system is used for detection, the imaging can be performed in two-dimensions or three-dimensions according to the probe used. For each eigenfrequency, corresponding RF signal sequences are correlated to obtain displacements. Each sequence of displacement images will then be Fourier transformed to extract stationary displacement maps which reveal the eigenmode corresponding to the tested resonance frequency. The images of the different eigenmodes can then be displayed on an output device 20 for study by the clinician. Optionally, the images can be segmented to automatically extract the geometry of the inclusion or heterogeneity.

The system and method of the present invention can also be used to characterize viscoelastic properties of confined structures, such as soft biological and industrial materials within or on a body, to enable the study of the viscosity and the elasticity of the confined structures. By 'soft' materials it is meant materials having a shear modulus generally between 10 Pa and 100 000 Pa, for example between 200 Pa and 20 000 Pa. The confined structure may be softer or harder than the body in or on which it is incorporated. In order to do so, simultaneously to the imaging step described above, an optimization step is performed to solve an inverse problem to assess the elasticity (stiffness) and viscosity of the heterogeneity. This may be done by using both measured and calculated (using a theoretical model) spectra. Obtaining the mechanical properties and geometry of the heterogeneity, the theoretical eigenmode images may be calculated and displayed for comparison.

Referring now to Figure 10, in an embodiment of a system for material rheology characterization, comprising a shear wave generation unit 12, an amplifier 40, a USB communication port 42, a processor 44, an acquisition card 46, a measured signal conditioning unit 48 and a monitor output 50, the sample 14 is placed into a fully identified and known material which is stable and whose material and mechanical properties are known. This material supports the transmission of shear waves. The knowledge of the surrounding medium mechanical properties permits the adaptation of the incident wave amplitude and the full control of the induction of resonance. Such a system may be provided as a portable device.

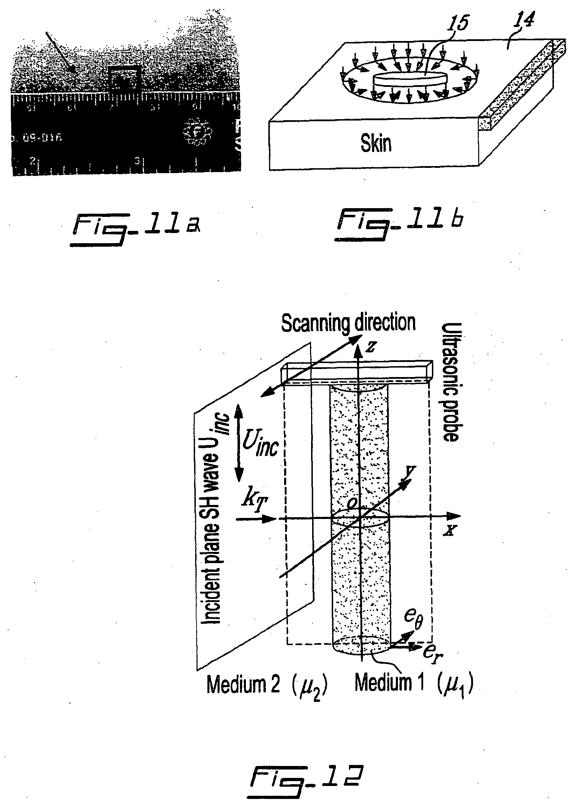

The present invention may be applied for the characterization of skin tumors, as shown for example in Figure 11a, taken from S.J. Kirkpatrick and R.K. Wang, "Imaging the mechanical stiffness of skin lesions by in vivo acousto-optical elastography", Optics Express, 14 (21), pp. 9770-9779, 2006. Figure 11b illustrates a system for inducing the resonance of such a skin tumor according to an embodiment of the present invention. The skin tumor is modeled in Figure 11 b by a thin disc 15 present at the surface of the skin 14. Resonance in the skin tumor 15 can be induced by producing surface waves (Rayleigh waves) by impacting the skin using rings or by producing waves directly in the plane of the tumor by imposing a movement of extension along a centered circle around the tumor (see arrows). In both cases, a frequency study allows the extraction of the eigenfrequencies of the tumor in-plane vibrations. The displacement measurements (to extract the resonance spectra and the various eigenmode images) can be performed using a high-frequency ultrasonic scanner equipped with a probe operating at a high frame rate.

The following are a few examples of the present invention.

Example 1

In the context of dynamic elastography, forcing the mechanical resonance of confined heterogeneities subjected to properly chosen incident shear waves can serve several objectives. A first objective is to improve the potential of dynamic elastography imaging to segment mechanically heterogeneous regions by maximizing the displacement contrast between the heterogeneity and its surrounding medium. A second objective is to propose a viscoelasticity characterization method based on the inclusion resonance properties. In the framework of elasticity imaging of cylindrical structures such as deep vein thrombi and, more generally, vascular pathologies, shear wave induced resonance of a circular cylindrical heterogeneity is experimentally investigated on a tissue phantom. It is shown that shear horizontal (SH) waves satisfy the physical conditions to induce resonance. Identification of the appropriate incident wave permits the formulation of an analytical model to simulate the heterogeneity-shear wave interaction and predict the resonance frequencies and eigenmodes. Experimental and theoretical results are compared and their contribution to dynamic elastography discussed.

As illustrated in Figure 12, the plane incident shear wave propagates following the X direction and is polarized following the cylinder axis along the Z direction. One can notice that these characteristics are those of a plane SH-wave. The system used is illustrated in Figure 3. Experiments were performed on a phantom (medium 2) made of 4.0% porcine skin gelatin and 3.0% agar powder (Sigma Chemical, Saint-Louis, MO, USA) dissolved in distilled water, containing a 5 mm radius circular cylindrical softer inclusion composed of 3.0% gelatin and 1.0% agar (Medium 1 mimicking a blood clot). Complex shear viscoelastic properties of Mediums 1 and 2, governed by the Hooke-Voigt law, were assessed on two different cubic square samples using harmonic plane shear waves at various frequencies for control measurements (see S. Catheline et al., "Measuring of viscoelastic properties of homogeneous soft solid using transient elastography: an inverse problem approach", J. Acoust. Soc. Am., 116 (6), pp. 3734-3741, 2004). The complex viscoelasticity $\mu_1$ and $\mu_2$ obtained for medium 1 and medium 2 were $(2700+0.05i\omega)$ Pa and $(17000+0.08i\omega)$ Pa, respectively, with relative assessment errors of $\pm$ 3.5% for the elasticity and $\pm$ 13.0% for the viscosity.

The shear waves were generated using the system of Figure 3 further provided with a temperature-controlled chamber, with a rigid vibrating plate 22 connected to a vibrator 24 (Brüel&Kjær, type 4810, Nærum, Denmark) and maintained in contact with the phantom (medium 2). The vibrator displacement was powered by a function generator 27 (Agilent, model 33250A, Palo Alto, CA, USA) and measured by an accelerometer 28 (Brüel&Kjær, type 4375, Nærum, Denmark). For a realistic comparison between experiments and theory, incident shear wave amplitudes of simulations were taken equal to those applied on the phantom (medium 2) and measured with the accelerometer 28. Radio frequency (RF) acoustic signals used to track vibrational motions within the phantom (medium 2) were acquired with a clinical 10 MHz array transducer connected to a Sonix RP scanner (Ultrasonix Medical Corporation, Bumaby, BC, Canada). By sequentially synchronizing probe acquisitions with the mechanical excitation (shear wave gating) and by using a retrospective reconstruction strategy, the frame rate reached 3850 images per second (a high frame rate is required to track SH vibrations). Displacements were calculated using a normalized cross-correlation algorithm applied on RF signals. Shear waves induced resonance elastography (SWIRE) measurements were performed in two steps.

First, displacement spectra produced by harmonic SH waves at frequencies f ranging from 70 Hz to 350 Hz (with $\Delta f$= 0.5MHz) were measured, at two different locations $M_1$ and $M_2$ within the inclusion. The objective was to extract the cylinder vibration eigenfrequencies corresponding to the mechanical configuration illustrated in Figures 1, 9 and 13. Tow ultrasonic beams, parallel to the axis z and crossing the plane $(o,x,y)$ at $M_1(-0.5mm,-1.15mm)$ and $M_2(-2.4mm,-1.15mm)$, were chosen to be sensitive to a maximum of eigenmodes. Indeed, as it will be shown later, the stationary displacement fields differ in amplitude from one eigenmode to another depending on their shapes. The second step consisted in imaging vibration eigenmodes corresponding to each measured eigenfrequency. A 3D scanning was performed by sequentially translating, following the Y axis, the ultrasonic probe to image 35 consecutive planes along a distance of $21.3mm$, Since the scattering problem did not depend on the Z coordinate, displacement fields were averaged, without filtering, following the elevational direction to get 2D displacement images. Applying a Fourier analysis to the temporal measured signals, displacement spectra and stationary displacement maps were obtained.

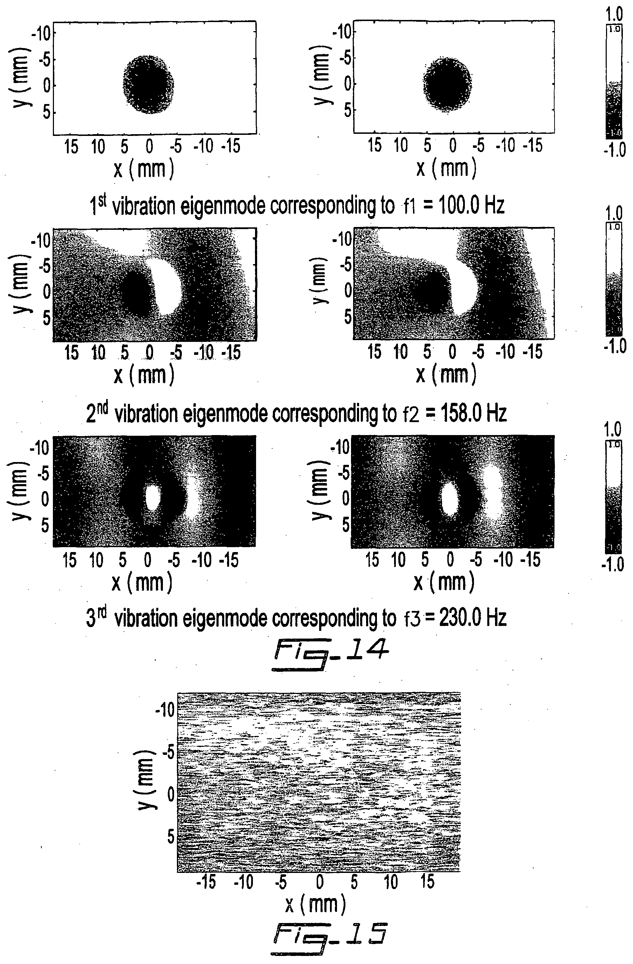

Figures 14a and 14b illustrate the measured displacement spectra, at the two positions $M_1$ and $M_2$, respectively. Three dominant frequencies clearly emerge: $f_1$=100$Hz$, $f_2$ =158$Hz$ and $f_3$ = 230 $Hz$. Amplification of displacements at these frequencies, particularly at position $M_2$ where eigenmodes are more contrasted, is characteristic of a resonance phenomenon.

Because the physical understanding of such resonance is important to explore its potential for dynamic elastography, an analytical model was developed to simulate the scattering of a SH wave (propagating along the $x$ axis) by a cylindrical circular heterogeneity embedded in a different material. Both media were assumed to be homogeneous, isotropic, and linear viscoelastic. Since the displacement field is purely transverse and parallel to the $z$ axis (see Figure 12), its divergence is equal to zero. In the frequency domain, the Navier differential equation, which governs the displacement field in both media (J.D. Achenbach, Wave Propagation in Elastic Solids, North-Holland, Amsterdam, 1973) becomes Helmholtz equation:

$$\mu_j(\Delta \mathbf{U}_j) + \rho_j \omega^2 \mathbf{U}_j = \mathbf{0} \quad \text{with} \quad j = \{1, 2\},$$

where $U_j = U_j(r, \theta)e_z$ is the stationary displacement field in a phase j, whereas $\rho_j$ and $\mu_j$ are the density and complex viscoelastic shear coefficient of the current phase, respectively. In the following, the wave number in each phase is defined by $k_{T_j} = \omega/c_{T_j}$ (where $c_{T_j}$ is the shear wave velocity). The Helmholtz equation can be solved in a cylindrical system of coordinates $(o, e_r, e_\theta)$ by mean of Bessel and trigonometric function series (P.M. Morse and H. Feshbach, Methods of Theoretical Physics, McGraw-Hill, New York, 1953, Chap. 11.2, p. 1376). Displacement in medium 2 is then a combination of the known incident plane wave and the scattered one. This latter, as well as the displacement within the inclusion, is expressed using series containing unknown coefficients $A_n$ and $B_n$ :

$$U_1 = \sum_{n=0}^{+\infty} A_n J_n(k_{T_1} r)\cos(n\theta)$$

$$U_2 = \sum_{n=0}^{+\infty} \left( B_n H_n^{(1)}(k_{T_2} r) + \varphi(\omega) \varepsilon_n (i)^n J_n(k_{T_2} r) \right) \cos(n\theta)$$

where $\varepsilon_n$ is the Neumann factor, $\varphi(\omega)$ is the incident wave amplitude, and $J_n(.)$ and $H_n^{(1)}(.)$ are the first kind Bessel and Hankel functions, respectively. The even angular dependence was chosen to satisfy the scattering symmetry conditions. Coefficients $A_n$ and $B_n$ ($n = 0,...,\infty$) were calculated by taking into account the continuity of displacement and normal stress at the cylinder boundary: $U_1 = U_2$ and $\sigma_{rz1} = \sigma_{rz2}$ (in $r = R$). Using the above displacement expressions and calculating normal stress at the boundary $R$, one obtains a system of two equations containing the infinite set of unknowns. The orthogonality property of trigonometric functions permits the separation of boundary conditions into an infinite set of systems of linear equations with respect to the order $n$:

$$\mathbf{T}_n \begin{pmatrix} A_n \\ B_n \end{pmatrix} = \mathbf{b}_n \quad \text{with} \quad r = R \quad \text{and} \quad n = 0,...,+\infty.$$

$\mathbf{T}_n$ and $\mathbf{b}_n$ are a matrix and a vector containing the $n^{th}$ order contribution of, respectively, scattered and incident fields to displacement and stress at the boundary. Solving the above linear system of equations for each order $n$ (until a truncature order N), permits the determination of the searched coefficients and, consequently the total stationary displacement field.

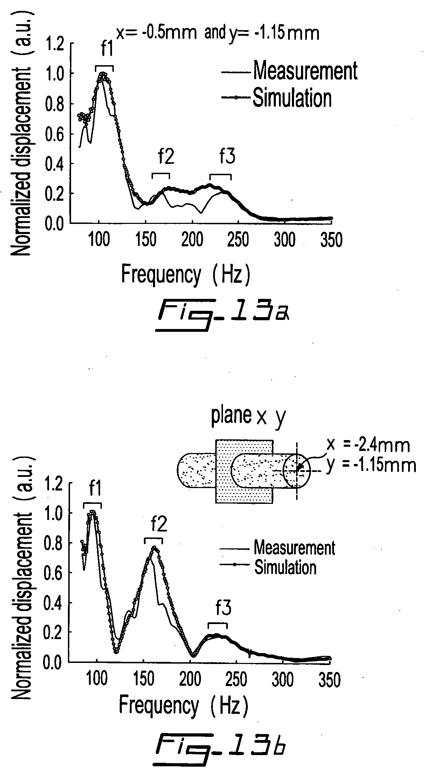

Theoretical displacement spectra at positions $M_1$ and $M_2$ were calculated using the measured viscoelastic and geometrical properties of both media. For a realistic comparison between experiment and theory, simulated incident SH waves had an amplitude spectrum equal to the one measured by the accelerometer. The comparative results in Figures 13 reveal a good superposition. Differences in amplitude and frequency can be explained by uncertainties on measured mechanical properties of media 1 and 2, accelerometer measurement error and that of the intercorrelation algorithm. However, one can conclude that the model is able to predict resonance frequencies and spectral shapes that are directly related to the media viscoelastic and geometrical properties.

Then the heterogeneous inclusion was excited, experimentally and theoretically, at the three identified eigenfrequencies to visualize the corresponding eigenmodes of vibration (Figures 14). Experimentally, the three incident waves were composed of 20 sinusoidal oscillations with frequencies of $f_1 = 100$ Hz, $f_2 = 158$ Hz and $f_3 = 230$ Hz.

As can be seen in the stationary displacement fields shown in Figures 14, the three eigenmodes appear clearly and a strong contrast exists between the inclusion displacements and those of the surrounding medium. If one aims to use elastography to identify confined inclusions, the resonance clearly permits an accurate segmentation of its boundary from stationary images, particularly for the first eigenmode, since this latter imposes to the whole cylinder an in-phase displacement along the z direction. The second eigenmode has the particularity to split the inclusion in two equivalent parts vibrating in opposition of phase. The apparition of three vibrating regions characterizes the third eigenmode: the inclusion center oscillates in opposition with its two adjacent regions. It appears, in Figures 14, that experimental eigenmodes (on the left) are well simulated by the model (on the right). This result, in addition to the well good superposition of spectra in Figures 13, permits to conclude to the existence of resonance eigenmodes and to the validity of the model for this cylindrical configuration.

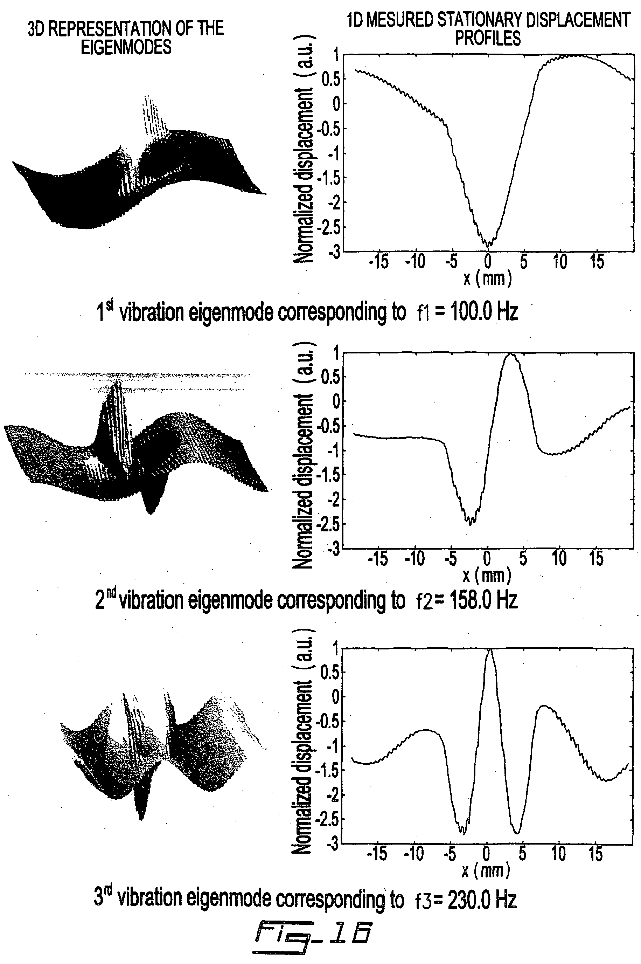

Figure 15 shows an echographic B-mode image of the phantom tested in the above experiments containing the soft circular inclusion (10.8 mm in diameter). The border of the inclusion is represented artificially in Figure 15 by a circle. In spite of the presence of a strong mechanical contrast between the inclusion and its surrounding medium, there does not appear any contrast in echogenicity. This emphasizes the need for the system and method of the present invention for identifying such inclusions. Figures 16 illustrates three-dimensional representations of the experimentally measured stationary eigenmodes (left) and the corresponding displacement profile measured along the X axis, crossing the cylinder diameter (right). Contrary to the echographic B-mode image (Figure 15), the inclusion appears clearly in the eigenmode images at the different inclusion resonances. This proves the potential of embodiments of the present invention to discriminate confined heterogeneities mechanically different from their environment. The method also allows the mechanical characterization of both heterogeneity and surrounding media since the resonance mechanism and its properties (eigenfrequencies, resonance spectra, etc.) depend on the constitution of the inclusion (e.g., the presence of a vessel wall surrounding the inclusion), its geometry, and mechanical viscoelastic properties.

Physically, the resonance phenomena are explained by the constructive combination of shear wavefronts within the inclusion and the absence of acoustical mode conversion affecting SH waves. For certain incident wavelengths, corresponding to the eigenfrequencies and related principally to the inclusion geometry and viscoelasticity, the constructive combination of refracted wavefronts into the inclusion allows the formation of standing waves and, consequently, the amplification of displacements.

Displacement eigenmodes presented in Figures 14 and 16 show the capacity of the present method and system to discriminate a heterogeneity from its background since resonances are confined. From a medical imaging point of view, in the absence of echogenicity contrast (as in the present case, see Figure 15), this allows to segment a different mechanical region directly from the eigenmode displacement maps. The mechanical resonance enhances the displacement signal to noise ratio and should, consequently, optimize the quality of dynamic elastography images. Further, from a mechanical point of view, the present system and method could serve to characterize viscoelasticity of confined heterogeneities by solving an inverse problem involving measured and simulated data. Indeed, the vibrational eigenfrequencies and the spectral displacement shapes (relative peak amplitudes, quality factor, etc.) of an inclusion are related to its geometry, viscoelasticity and mechanical contrast with respect to the surrounding medium. More generally, since accessible resonance frequencies cover a relatively large spectral range, this characterization method could permit determining the viscoelastic behaviour law of studied tissues. Consequently, the present method and system can also serve to design a rheological measurement apparatus to characterize soft materials (and their mechanical evolution) for both academic and industrial applications. Due to enhancement of contrast at resonances, the clear identification of eigenfrequencies, and the ability to formulate and solve an exact inverse problem (based on a fast analytical model), this characterization approach could represent a more robust, controllable, and reliable alternative to the classical devices.

Analytical study of the scattering of plane shear waves by a viscoelastic sphere or by an elliptical cylinder both embedded in a viscoelastic surrounding medium were further extended. It appears that the theoretical basis is similar in all cases. Both types of inclusions (sphere or elliptical cylinder) and surrounding media are assumed to be viscoelastic:

$$G = G' + iG''$$

where G is the complex shear viscoleastic modulus, $G'$(Pa) is the shear modulus and $G''$(Pa) corresponds to the viscous part of G.

Displacements fields in both media are governed by the Navier differential equation, which, assuming the harmonic case (*i.e.* temporal dependence of displacements in $e^{i\omega t}$), can be written as follows:

$$\rho_j \omega^2 \mathbf{U}_j - \left(\lambda_j + \mu_j\right)\nabla\left(\nabla.\mathbf{U}_j\right) - \mu_j \nabla^2 \mathbf{U}_j = 0 ; \quad j = 1,2$$

with *j*=1,2 indicates inclusion or surrounding medium, respectively. $\mathbf{U}_j$ designates the 2D or 3D vector field of displacements in medium *j*, $(\lambda_j, \mu_j)$ are Lamé material parameters and $\rho_j$ is the density.

Applying a potential decomposition to $\mathbf{U}_j$, in terms of compressional and shear potentials, allows to rewrite the above equation as two Helmholtz equations, typically of the form:

$$\nabla^2 \Psi + k_s \Psi = 0$$

$$\nabla^2 \Phi + k_c \Phi = 0$$

where $k_s = \omega \sqrt{\dfrac{\mu}{\rho}}$ and $k_c = \omega \sqrt{\dfrac{(\lambda + 2\mu)}{\rho}}$ are wave vectors of compressional and shear waves, respectively. $\Phi$ is a scalar potential (compressionnal wave) and $\Psi$ a vector potential (shear wave).

Analytic solutions of the two Helmholtz equations above depend on the coordinates system. In spherical coordinates, solutions are expressed in terms of infinite series of vector spherical harmonics (implying Legendre polynomials) whereas infinite series of radial and angular Mathieu functions are used in elliptical coordinates. In both systems of coordinates, potentials can be written as:

$$\Phi = \sum_{n=0}^{\infty} \sum_{i} A_{in} f_{in}(\mathbf{M}, k_c)$$

$$\Psi = \sum_{n=0}^{\infty} \sum_{i} B_{in} g_{in}(\mathbf{M}, k_s)$$

where $A_{in}$, $B_{in}$ are unknown coefficients to be determined which depend on frequency, shape of the inclusion and boundary conditions. $f_{in}(M,k_c), g_{in}(M,k_s)$ are combinations of spherical harmonics (in spherical coordinates) or Mathieu functions (in elliptic cylindrical coordinates). Both functions depend on position vector M in each coordinate system, and wave vectors $k_c$, $k_s$. Index i indicates Mathieu functions parity (elliptical case), or in the spherical case, the nature of the solution's component (shear or compression). It is important to note that in the spherical case, a third potential $\Xi$ is necessary to express the second type of shear wave and has the same form as $\Psi$.

Resolving the two Helmholtz equations above consists in computing unknown coefficients. This is achieved by taking into account the boundary conditions, which in the case of a viscoelastic inclusion embedded in a viscoelastic medium are the continuity of displacements and stresses components at the boundary of the inclusion.

Once the problem has been explicitly expressed and solved for suitable boundary conditions, displacements may therefore be computed in both media, at any frequency of the incident wave and any incidence angle. In order to assess frequency dependence of displacements inside the inclusion, spectra of displacements are computed for both geometries, at discrete points inside the inclusion, and displacements maps are established for discrete frequencies.

In the case of the spherical inclusion, as illustrated in Figure 18a, the origin of the coordinates system *(x,y,z)* is taken at the center of the spherical inclusion. Incident plane shear wave propagates along the z-axis, and is polarized along the x-axis. Since the sphere geometry is symmetrical, the incidence angle can take any value. The inclusion (medium 1) is a sphere of radius 6.0 mm, embedded in an infinite surrounding medium (medium 2). Both materials are governed by a Hooke-Voigt model and viscoelastic parameters are respectively: $G_1 = 2.4.10^3 + i\omega 0.056$, and $G_2 = 17.10^3 + i\omega 0.7$, (see the equation for the complex shear viscoleastic modulus above). Spectrum of displacements (Figure 18b) highlights resonance frequencies which are characterized by higher displacement amplitudes compared to other frequencies. The first three resonance frequencies can be identified *as $f_1$ =160.3Hz, $f_2$ = 205.4Hz, $f_3$ = 259.2Hz.* For a stiffer inclusion embedded in an identical surrounding medium, these particular frequencies are shifted to higher frequencies. In a more general way, resonance frequencies increase with elasticity. Displacement maps corresponding respectively to $f_1, f_2, f_3$.

Figures 18c, 18d and 18e allow visualizing the sphere eigenmodes of vibration compared to the displacements in the surrounding medium. The first displacement map (Figure 18c) is computed at $f_1$ =160.3Hz in the plane (O,y,z). Inside the sphere, displacements induced by the incident wave present a characteristic pattern of two distinct zones, parallel to the incident wave polarization, moving in phase opposition, and separated approximatively at the middle of the diameter. As shown by spectrum in Figure 18b, amplitude of displacements in the inclusion increases at resonance frequencies. These two characteristics allow a better segmentation of the inclusion. Displacement maps corresponding to $f_2$ = 205.4Hz in the plane (O,y,z) (Figure 18d) present a second eigenmode of vibration, which consists in three distinct zones where two adjacent parts moving in opposition of phase. Amplitude of displacements is higher in the middle of the inclusion contrary to the precedent eigenmode in which the center of the inclusion defines the separation of the two moving zones. The third eigenmode, computed at $f_3$ = 259.2Hz in the plane (O,y,z) consists in four distinct parallel zones of displacements, where two adjacent zones moving in phase opposition (Figure 18e).

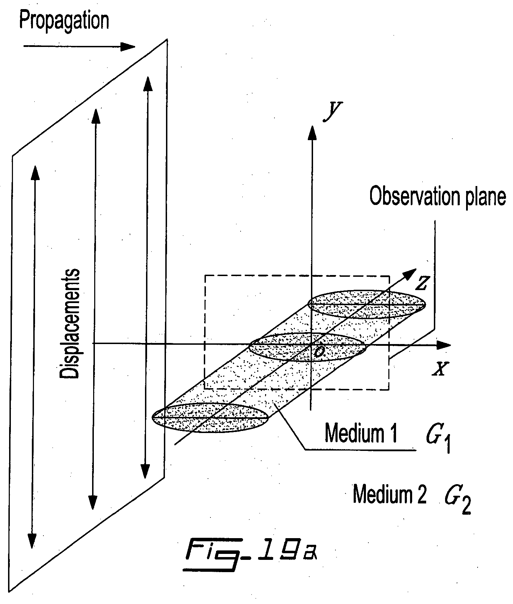

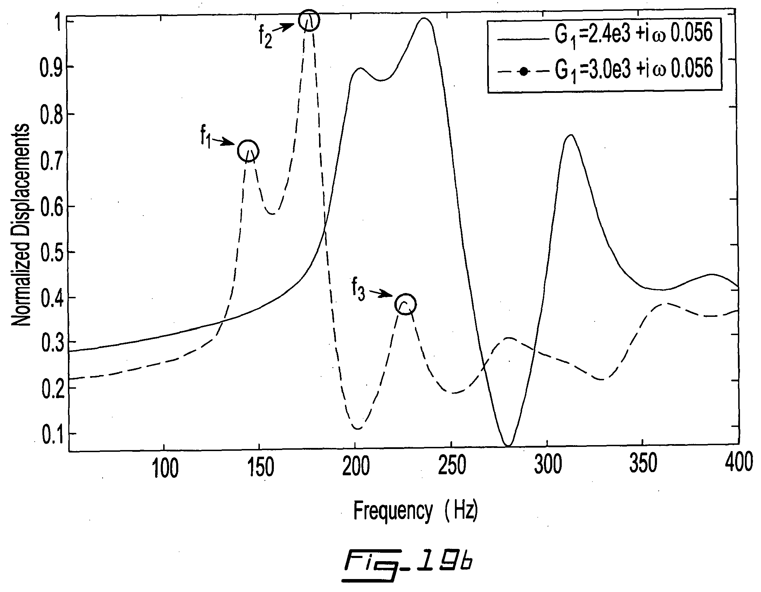

In the case elliptic cylindrical inclusion illustrated in Figure 19a, the cylinder axis defines the z-axis, whereas major and minor axes of the elliptical cross-section are along x-axis and y-axis, respectively. Incident plane shear wave propagates along x-axis and is polarized along y-axis. Such polarization is called SV-wave for Shear Vertically polarized wave. The problem is invariant by translation along the z-axis and therefore can be reduced to a 2-D problem in the xy-plane. Incidence angle, as well as polarization, in the xy-plane can take any value. As previously defined in the spherical case, inclusion and surrounding medium are denoted medium 1 and medium 2, respectively. Spectrum of displacements is computed from 50Hz to 400 Hz, in the xy-plane at point (*1mm, 1mm*), for an incident shear wave propagating along the x-axis, polarized along the y-axis, for two values of $G_1$, defined following Hooke-Voigt's model: $G_1 = 1.2.10^3 + i\omega 0.056$ and $G_1 = 2.4.10^3 + i\omega 0.056$ (see Figure 19b).

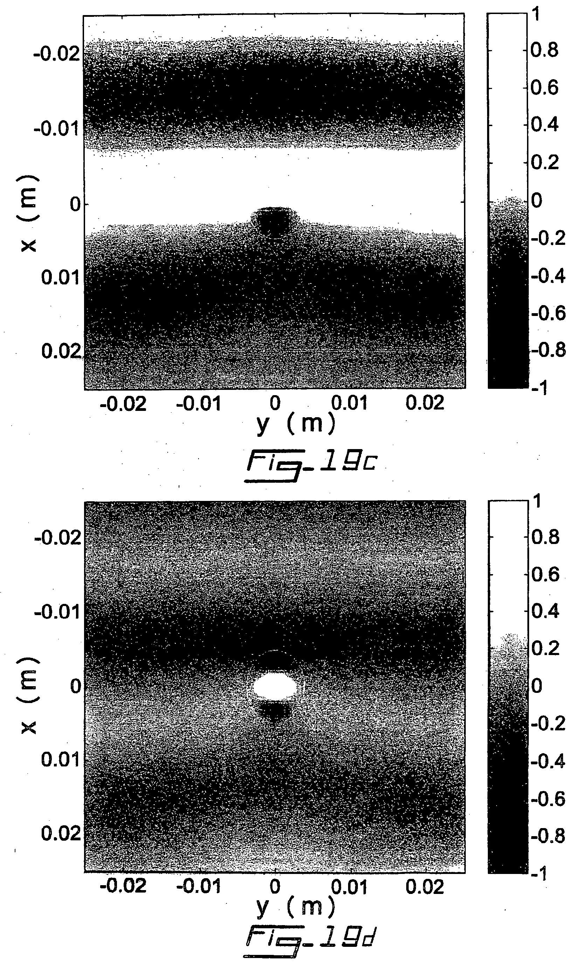

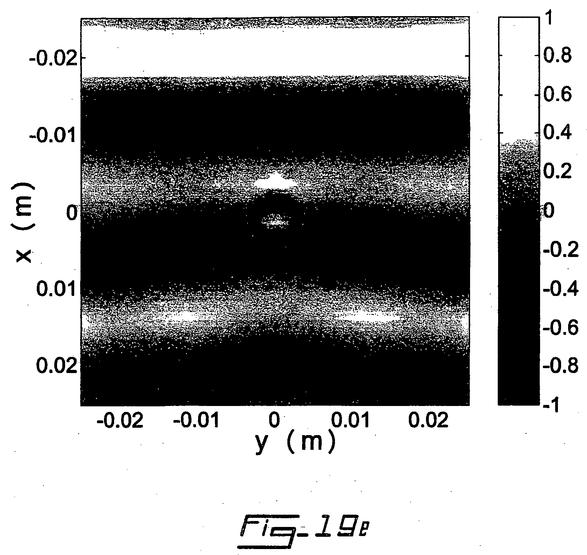

As in the spherical case, amplitude of displacements is enhanced for some discrete frequencies (eigenfrequencies). For an identical surrounding medium, eigenfrequencies increase with elasticity of the inclusion. Displacement maps are then computed at $f_1 = 146.1 Hz$, $f_2 = 178.6$ Hz, et $f_3 = 227.3$ Hz (see Figures 19c, 19d and 19e respectively). Displacements patterns inside the elliptical inclusion at resonance frequencies (*i.e.* eigenmodes of vibration) present the same characteristics as those observed in the spherical case. Indeed, cross section of the cylinder is subdivided in two, three and four distinct parts respectively at $f_1$, $f_2$, $f_3$. These eigenmodes also allow a visual segmentation of the inclusion since amplitude of displacements increases at resonance frequencies.

Thus, resonance of spherical and elliptic cylindrical inclusions induced by shear waves of different orientations has been analytically modeled. Eigenmodes visualized at eigenfrequencies are characterized by an enhancement of displacement amplitude in the inclusion compared to those observed at other arbitrary frequencies. This property is useful to segment pathologies with low ultrasonic impedance contrast with the surrounding medium (standard sonography limitation). Numerical methods such as Finite Elements Method (FEM), Finite Difference Method (FDM) allow to observe same eigenmodes in both geometries. However, analytical methods take advantage of low time computation, and furthermore, allow an inverse problem approach. This method consists in formulating an optimization algorithm dedicated to determinate viscoelastic parameters from resonance frequencies and geometries. This approach is reliable since it has been shown that resonance frequencies depend on the shape and mechanical properties of the inclusion, therefore constituting a relevant parameter in a viscoelastic characterization goal. Other analytical methods such as T-matrix method (TMM) or Helmholtz integral equation method (HIEM) can also be used to model scattering of plane shear waves by such geometries.

Hence, inducing resonance phenomenon into confined pathologies according to the present invention allows assessing enhanced segmentation in complement to standard sonography, and spectrum of displacements provides information about viscoelastic properties of the propagating medium.

Clinically, shear waves can be generated externally in biological tissues by using a vibrating plate, a spherical cap, a wire, or internally, using ultrasonic radiation force method. Shear wave propagation in tissues is then assessed ultrasonically. For example, in a breast imaging context, establishing a spectrum of displacements inside a nodule over a frequency range can provide information about its viscoelastic parameters. A suitable use of transient waves properties should limit the number of acquisitions. If resonance frequencies are detected, displacement maps acquired at those frequencies allow an enhanced segmentation, by visualizing eigenmodes of vibration. Finally, an optimization algorithm can provide quantitative values of viscoelastic parameters, using an inverse problem approach.

Example 2

In order to characterize the viscoelasticity of a material sample contained as a confined inclusion in a known homogeneous material, the experimental set up and methodology of Example 1 was adapted to perform soft material rheology characterization. Firstly, the resonance frequencies and the resonance spectrum of the material sample were measured. Since the eigenfrequencies and the spectral response of the sample depend on its geometry and viscoelastic properties, one can solve an inverse problem, involving a theoretical model, to assess the sample viscoelasticity. The theoretical model used to solve the inverse problem can be parameterized by the known geometrical properties of the tested sample and the mechanical properties of the known surrounding soft material.

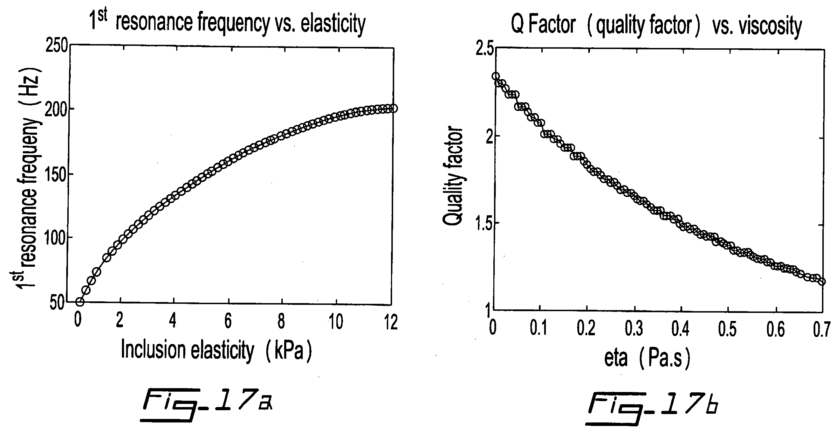

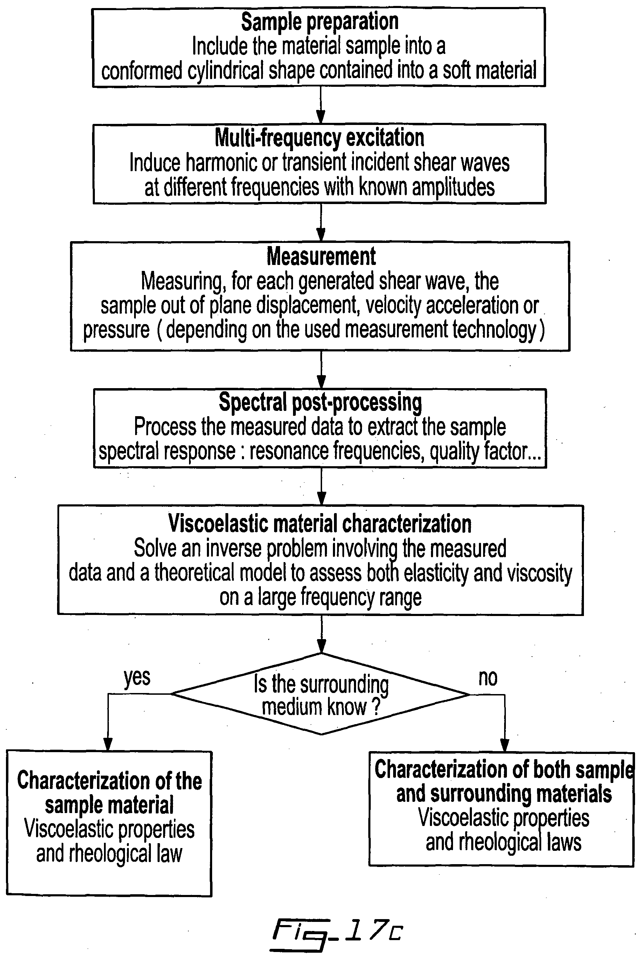

The evolution of the first resonance frequency and of the quality factor with respect to the shear elasticity and viscosity are plotted in Figures 17a and 17b, respectively. Figure 17c illustrates a flow chart of a method for solving the inverse problem and assessing the viscoelasticity values. The first step comprises preparing the sample. The sample to be characterized is contained in a soft material in a conformed cylindrical volume. Other conformed shapes may also be possible. A multi-frequency excitation process is then performed by sending harmonic or transient shear horizontal waves to the sample with different frequencies and known amplitudes as described for embodiments of the invention. For each frequency excitation, the sample response (i.e. the out of plane induced displacement, velocity or pressure) is measured using an adapted technology (e.g. ultrasound, laser, microphone, etc.). The measured response is then processed by a spectral analysis in order to extract the resonances frequencies of the sample (and, possibly, the eigenmode images), the spectral quality factor, the displacement amplitudes, etc. This experimental information will serve to solve an inverse problem involving an adapted theoretical model (depending on the sample geometry) to assess the real and imaginary parts of the viscoelasticity module of both sample and surrounding media (if the latter is unknown). The inverse problem formulation depends on the desired rheological characterization. For example, when both the inclusion and surrounding media have to be characterized, the algorithm should use experimental data measured in both media. To solve such an inverse problem, one can formulate it following, for example, a least square method which could involve a minimization algorithm to assess the viscoelastic parameters. Knowing the frequency dependence of viscoelasticity, the rheological characterization post-processing can find the best fitting rheological model among a predefined set of rheological models (e.g. Voigt, Maxwell, Jeffreys, fractional Voigt, Prony series, etc.).

The quality factor of a spectrum is directly related to the material viscosity around a certain frequency. The more viscous the material is, the more the spectrum is spread out (or opened) and inversely. The quality factor is thus a good candidate to assess the viscosity of the resonant sample. The results of Figures 17 show a strong sensitivity of the first eigenfrequency and quality factor to the inclusion elasticity (or stiffness) and viscosity. It is thus possible to exploit the measured eigenfrequencies and the shape of the spectra to estimate the viscoelasticity of the inclusion. More generally, since accessible resonance frequencies cover a relatively large spectral range, this characterization method can permit the determination of the viscoelastic behavior law (or rheological law) of the studied material sample. This aspect of the present invention may be used to characterize experimentally and without any a priori, the rheological model governing the material mechanical behavior among different existing models (such as the Voigt, Kelvin-Voigt fractional derivative, Maxwell, Jeffreys, Zener, fractional Zener and Prony series models, etc).

Example 3

Applied to vascular dynamic elastography, and particularly to the problem of mechanical characterization of deep vein thrombi, or to the problem of mechanical characterization of elliptical cancerous lesions, this example presents shear-wave induced resonance of general shaped elliptical heterogeneities, according to an embodiment of the present invention.

The blood clot or lesion was modeled as an elliptic cylinder. Both inclusion and surrounding media were assumed to be isotropic, homogenous and viscoelastic. The incident plane shear wave was polarized in the Z direction (i.e., following the cylinder axis). Solving the governing differential equation (Helmholtz wave equation) in elliptical coordinates lead to the expression of the total displacement field as series of Mathieu functions (A.R. Hadj Henni and C. Bacon, In-plane vibration of thin elliptic plates submitted to uniform pulsed microwave irradiations", Journal of Sound and Vibration, 299, pp. 298-313, 2007) with unknown coefficients. Taking into account boundary conditions permitted to determine these coefficients and, consequently, both scattered and refracted displacement fields. The computation of the displacement spectrum at a discrete position within the inclusion allowed to observe resonance frequencies. By calculating the total displacement fields corresponding to these eigenfrequencies, the shapes of the eigenmodes could be obtained.

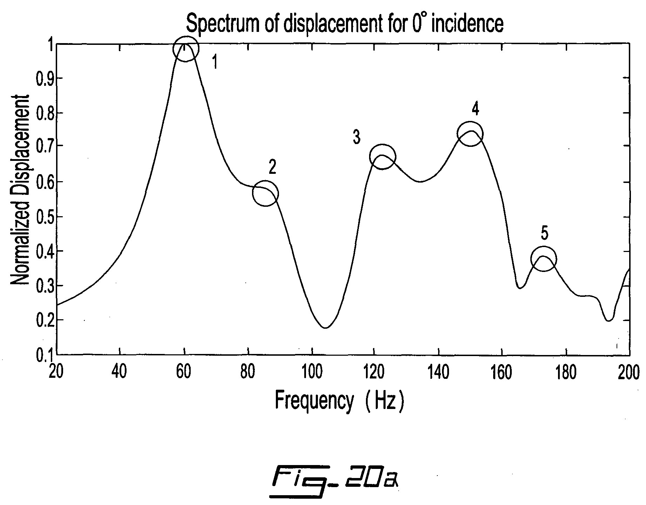

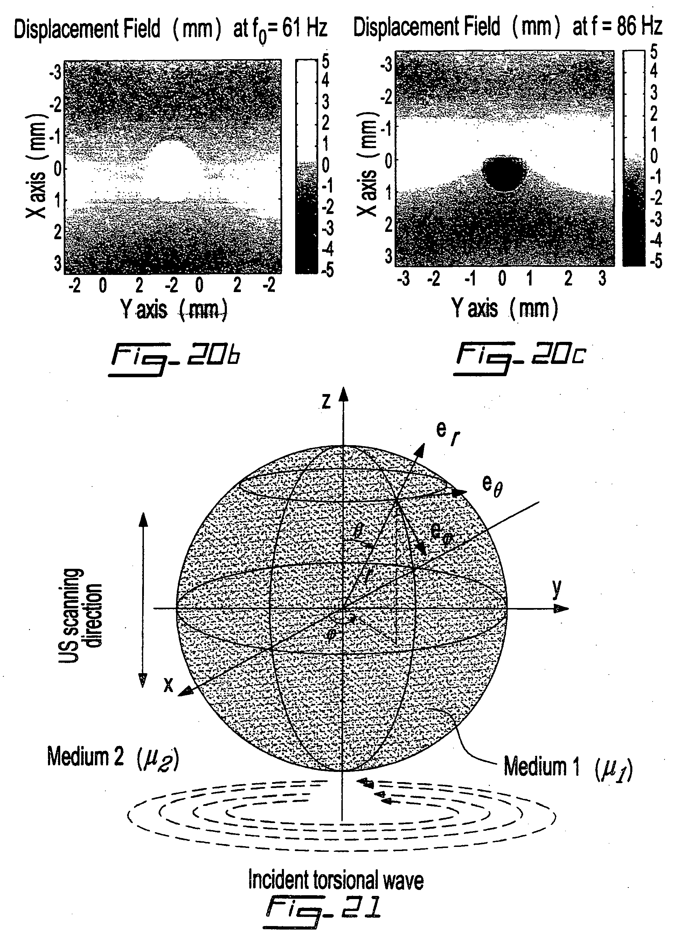

The spectrum of displacements exhibiting eigenfrequencies, which define vibrational resonances of the heterogeneity, is shown in Figure 20a. Figures 20b and 20c present two displacements fields: a first vibration mode at 61 Hz (20b) and a second vibration mode at 86 Hz (20c). It appears that at the first resonance frequency, the whole inclusion was vibrating in phase with the incident wave. In the second mode, displacement regions vibrating in opposite directions were observed within the inclusion on both sides of the minor axis. For both eigenfrequencies, the inclusion boundary could be clearly delimited in the stationary displacement images (at resonances).

A semi-analytical model of plane shear-wave scattering by elliptical cylinders has been developed. The system and method of the present invention permitted the determination of resonance frequencies of confined inclusions, as seen in the spectrum and displacement fields of Figures 20. Embodiments of the present invention provide relevant advantages for dynamic elastography such as a fast segmentation due to the increased contrast between the inclusion and surrounding media, and can allow tissue characterization (viscoelastic properties) by solving, for example, an inverse problem.

Example 4

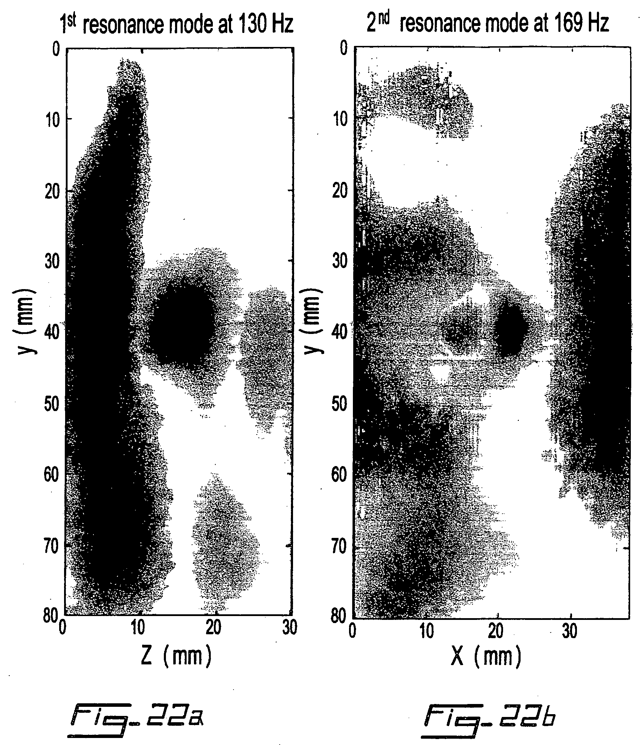

The in vitro experiment of example 1 was repeated for a spherical inclusion using the system of Figures 4. The surrounding medium, made of 4% gelatin and 3% agar, contained a spherical heterogeneity composed of 3% gelatin and 1% agar. The spherical heterogeneity had a diameter of 14.0 mm $\pm$ 1 mm. When a torsional incident shear wave is scattered by a spherical inclusion, embedded in a soft material, mode conversions do not occur and both diffracted and refracted elastic waves are purely circumferential. The constructive combination of the refracted shear waves into the heterogeneity and the absence of mode conversions induce the resonance of the sphere. A plane torsional incident wave, polarized in the circumferential direction, was propagated following the z direction, as illustrated in Figures 21. In the present example, displacements were measured along the y-axis direction using a 10 MHz ultrasound probe of the Sonix RP scanner (see system of Figure 4). A frequency study revealed that two resonances occurred at frequencies of 130.0 Hz and 169.0 Hz. Figures 22a and 22b represent the two-dimensional stationary displacement fields (imaged following a plane perpendicular to the z propagation axis) obtained at the two identified resonance frequencies. As in the cylinder case, the two resonance eigenmodes are clearly identified. In addition, the sphere boundary appears clearly in the stationary images. This segmentation properly shows the ability of the present method to discriminate heterogeneous regions directly from displacement maps. This property, coupled to a viscoelastic characterization method, is of great interest for medical elastography imaging of confined pathologies or organs such as breast tumors, prostate, lung tumor, etc.

Example 5

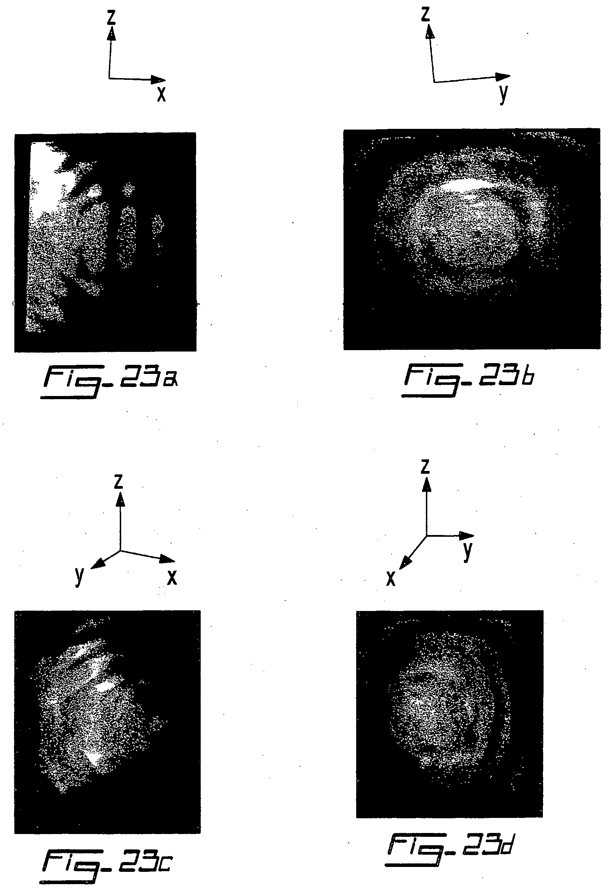

The efficiency of the focusing method discussed in relation to Figure 6 was tested in a homogeneous phantom made of 4% gelatin and 3% agar (in proportion of water weight). A hollow spherical cap (obtained form a sphere with a diameter equal to 60.0 mm) was used to focus the torsional shear waves. The center of the original sphere was located at 15.0 mm from the circular edge of the spherical cap. Different three-dimensional views of focalized shear waves into the homogenous phantom are illustrated in Figures 23a-23d. One can observe the convergence of the generated torsional waves to the center region of the spherical cap.

Example 6

Shear wave induced resonance elastography (SWIRE) has also been applied to image the resonance of confined spherical heterogeneities. When a circumferential torsional incident shear wave is scattered by a spherical inclusion, embedded in a soft material, it does not produce mode conversions and both diffracted and refracted elastic waves are purely circumferential. Let us assume a plane torsional incident wave. In a spherical system of coordinates $(o, e_r, e_\theta, e_\varphi)$, this kind of wave is polarized in the circumferential direction (following the tangential unit vector $e_\varphi$ as represented in Figure 22) and propagating following the z axis as represented in Figure 21. The materials of inclusion (medium 1) and that of surrounding tissue (medium 2) are homogeneous, isotropic, and linear viscoelastic. Since displacement has only one non-zero component following the circumferential unit vector $e_\varphi$, the Navier differential equation, which governs the displacement field in both media (J.D. Achenbach, Wave Propagation in Elastic Solids, North-Holland, Amsterdam, 1973, Chap. 2, p. 55.), becomes a Helmholtz one. In the frequency domain this latter is expressed as:

$$\mu_j(\Delta \mathbf{U}_j) + \rho_j \omega^2 \mathbf{U}_j = \mathbf{0}$$

where $j = \{1,2\}$, $\mathbf{U}_j = U_j(r,\theta)e_\varphi$, is the stationary displacement field in medium j, whereas $\rho_j$ and $\mu_j$ are the density and complex viscoelastic shear coefficient of the current medium, respectively. In the following, the wave number in each medium is defined by $k_{T_j} = \omega/c_{T_j}$ (where $C_{T_j}$ is the shear wave velocity). The Navier differential equation above can be solved in a spherical system of coordinates by mean of spherical Bessel functions and associated Legendre function product series (L. Knopoff, "Scattering of shear waves by spherical obstacles", Geophysics, 24, pp. 209-219, 1959; F. Schwab, "Scattering of shear waves by small transeismic obstacles", Geophysics 30, pp. 24-31, 1965). In the surrounding medium, the resulting displacement field is a combination of the known incident wave and the scattered one. This latter and the refracted torsional wave can be expressed using series containing unknown coefficients $A_n$ and $B_n$ :

$$U_1 = \sum_{n=1}^{+\infty} A_n j_n(k_{T_1} r) P_n^1(\cos\theta)$$

$$U_2 = \sum_{n=1}^{+\infty} \left( \varphi(\omega) i^n \frac{2n+1}{n(n+1)} j_n(k_{T_2} r) + B_n h_n^{(1)}(k_{T_2} r) \right) P_n^1(\cos\theta)$$

where $j_n(.)$ and $j_n(.)$ are the first kind spherical Bessel and Hankel functions, respectively, $P_n^1(.)$ is the associated Legendre function and $\varphi(\omega)$ is the incident wave amplitude. Coefficients $A_n$ and $B_n$ are calculated by taking into account the continuity of displacement and normal stress at the boundary of the spherical heterogeneity.

Using the displacements of the above equation and expressing the normal stress at $r = R$, one obtains a system of two equations containing the infinite set of unknowns. The orthogonality property of associated Legendre functions permits to separate, with respect to the order $n$, this system into an infinite set of systems of linear equations of two unknowns:

$$\mathbf{T}_n \begin{pmatrix} A_n \\ B_n \end{pmatrix} = \mathbf{b}_n \quad , \quad r = R \text{ and } n = 0, \ldots, +\infty \,.$$

$\mathbf{T}_n$ and $\mathbf{b}_n$ are a ($2 \times 2$) matrix and a vector containing the $n^{th}$ order contribution of the scattered and incident fields to displacement and stress at the boundary. Solving the above equation for each order $n$ (until a truncature order N), permits to determine the researched coefficients and, from the series $U_1$ and $U_2$ above, the total three-dimensional stationary displacement field.

To fix the parameters of the model, preliminary experiments were conducted on an agargelatin phantom containing a 7.0 mm radius spherical inclusion made of a softer agar-gelatin material. Here agar particles play the role of acoustical scatterers of ultrasound. Complex shear viscoelastic properties of both media, $\mu_1$ (complex viscoelasticity of the inclusion) and $\mu_2$ (complex viscoelasticity of the surrounding medium), were determined experimentally as described earlier and were (2700+0.05i$\omega$) Pa and (17000+0.7i$\omega$) Pa, respectively.

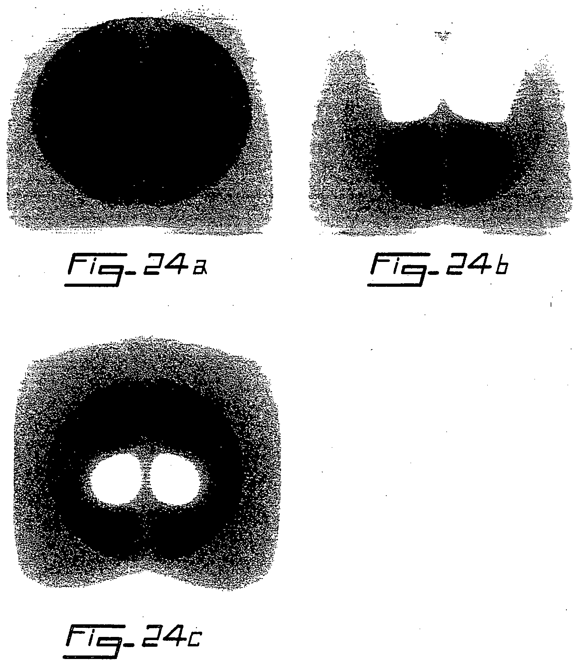

The first three eigenmodes of a spherical heterogeneity were calculated according to the theoretical model corresponding to the spherical geometry of Figure 21 (where R = 7.0 mm, $\mu_1$ = 2700 +0.05i$\omega$ in medium 1 and $\mu_2$ = 17000 +0.7i$\omega$ in medium 2). Figures 24a-24c illustrate the theoretical torsional displacement fields corresponding to the three first eigenmodes obtained at 140.0 Hz, 183.0 Hz and 225.0 Hz. A quarter of the sphere has been removed from the displays to observe displacement fields deeply into the heterogeneity. A transparency has also been applied to the images in order to show the inner displacement field.

Example 7

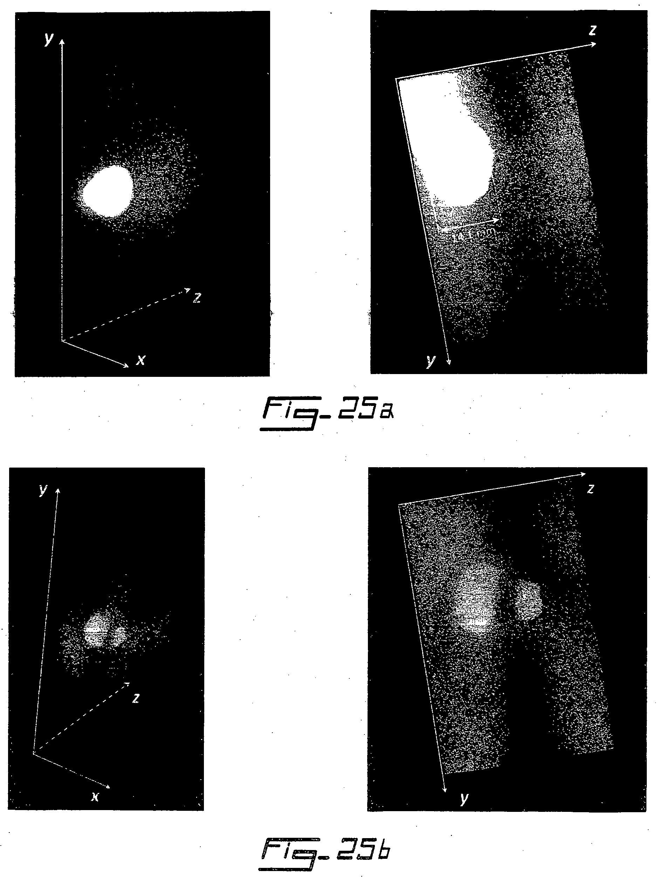

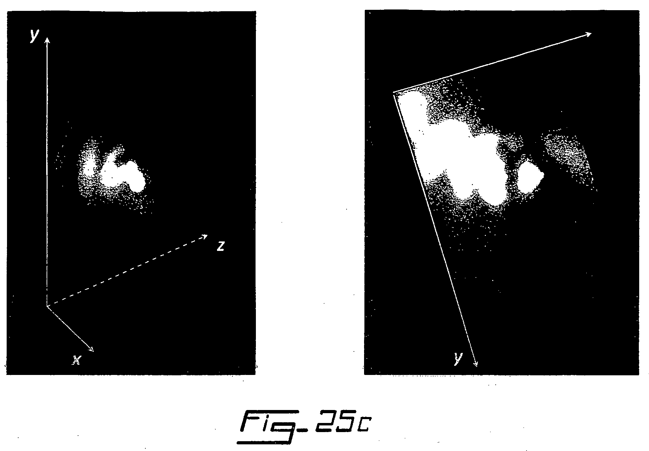

The theoretical model described in Example 6 was validated experimentally in a phantom including a spherical inclusion by using the focalized torsional shear-wave strategy illustrated in Figure 6 and displayed experimentally in Figures 25. The agar-gelatin phantom contained a 7.0 mm radius spherical inclusion made of a softer agar-gelatin material. Agar particles scattered ultrasound and allowed the detection of shear-induced displacements within the phantom. The complex shear viscoelastic properties of both media, $\mu_1$ (complex viscoelasticity of the inclusion) and $\mu_2$ (complex viscoelasticity of the surrounding medium), were estimated at (2700+0.05i$\omega$) Pa and (17000+0.08i$\omega$) Pa, respectively. The eigenmode images of the inclusion are illustrated in Figures 25 a, b and c for the three first resonance frequencies 120.0 Hz, 176.0 Hz and 261.0 Hz, respectively. One can observe that the spherical heterogeneity is clearly identified from the images.

As people in the art will now be in a position to appreciate, the present invention provides a novel approach to dynamic elastography and material characterization.

By means of embodiments of the present invention, the mechanical resonance of a confined mechanical heterogeneity or inclusion within a body is induced in order to obtain a good quality elastographic image of the heterogeneity and to achieve its mechanical characterization. By heterogeneity it is meant a part of the body which has different mechanical properties from those of the body itself. A confined heterogeneity is a heterogeneity of finite dimensions contained in a limited three-dimensional (3D) or two-dimensional (2D) space.

By means of embodiments of the present invention, certain localised pathologies on elastographic images can be better segmented and their mechanical properties more accurately determined. The mechanical properties of pathological heterogeneities can be reliable indicators for diagnosis of certain disease states. Embodiments of the present invention are particularly well suited, although not limited, to the diagnosis of pathological heterogeneities such as breast, prostate and skin tumors, venous thrombosis, and other abnormalities of tissues.

The present system allows inducing resonance of a heterogeneity within a body, the system comprising a shear wave generator arranged to apply mechanical shear waves to the body to induce resonance of the heterogeneity. For a heterogeneity having a longitudinal axis, the shear wave generator is arranged to apply mechanical shear waves parallel to the longitudinal axis of the heterogeneity. For a substantially spherical heterogeneity, the shear wave generator is arranged to apply mechanical torsional shear waves following a circumferential direction of the heterogeneity.

The present method for elastography of a heterogeneity within a body comprises applying mechanical shear waves to the body to induce resonance of the heterogeneity. For a heterogeneity having a longitudinal axis, mechanical shear waves are applied substantially parallel to the longitudinal axis of the heterogeneity. For a substantially spherical heterogeneity, the mechanical shear waves are applied substantially circumferentially to the heterogeneity.

The present invention provides a non-destructive and non-invasive in vivo and in vitro tool which can be used to evaluate in situ and ex-vivo samples.

The present invention allows increasing the mechanical response of a heterogeneity, and therefore its visualization and its mechanical characterization, by the induction of resonance through shear wave diffraction. The border or borders of a heterogeneity are defined in a precise way even when the heterogeneity does not present a contrast in echogenicity with its surrounding medium, or has a low contrast. The absolute mechanical properties (e.g., displacements, viscoelasticity, eigenmode of resonance, etc.) of the heterogeneity are precisely characterized.

Pathologies can be mechanically characterized and segmented visually and possibly numerically by using segmentation methods (e.g., thresholding).

Moreover, the present method and system are independent from the source of shear wave. The shear wave can be produced by any shape or configuration of a contact surface such as a vibrating plate, a cylindrical plate, a circular ring, a spherical cap, half (or part of) hollowed cylinder, a wire, or by a radiation force, etc.

The present method and system can be coupled to imaging methods used in dynamic elastography of materials, e.g. ultrasound, magnetic resonance imaging (MRI), Doppler techniques, acousto-optical methods, optical methods, or any other modalities that can track tissue motion.

The present method and system can be coupled to imaging methods of dynamic elastography of materials (e.g., impulse elastography, sonoelastography, magnetic resonance elastography, radiation force dynamic elastography, supersonic radiation force dynamic elastography, acousto-optic elastography, optical elastography, etc).

The present method and system can be integrated into medical or non medical commercial imaging scanners equipped with a dynamic elastography unit.

The method and system of the invention when used with a mono-element transducer in transient mode can provide very fast mechanical characterization data of a heterogeneity which can have application as a device for the in vitro characterization of biological and industrial materials.

The method and system of the invention can also serve to characterize the rheology of any soft material used, for example, in food, chemistry, pharmacy and material industries.

The method and system of the invention can be used to monitor sol-gel transition, quality control and rheological characterization of soft and viscous materials during an industrial process (online, i.e. in situ conditions) or for laboratory studies;

An apparatus according to an embodiment of the present invention for rheological characterization of materials can use any non-contact vibration measurement technology, for example, ultrasound, laser probe, high sensitivity microphone, high sensitivity camera, etc.

The method and system of the invention can be interactive in order to make it possible for the user, such as a clinician, to monitor the mechanical properties of materials in real-time.

The method and system of the invention can be used for therapeutic intervention of diseased tissues by inducing complex eigenmodes of resonance of said tissues and mechanotransduction effects.

Embodiments of the present invention can be used for imaging and characterizing heterogeneities, either partially or fully confined within a body, made up of a material softer or harder than the surrounding material. Applications include elastographic imaging of localised tumors and possibly, with a high frequency scanner, the characterization of skin tumors.

Other applications include the imaging and characterization of venous thrombosis and atherosclerosis. The present system and method may be used to image and characterize the mechanics (elasticity and viscosity) of the thrombus during its formation and evolution within blood vessels. Also, the composition of the thrombus can be studied in response to the administered medication using the present invention.

Other applications include the imaging and characterization of atherosclerosis. Atherosclerosis is defined by a morphological and mechanical change of the wall of certain arteries (e.g.. aorta, carotid, iliac and femoral arteries, etc). The present invention can be applied to obtain a segmented chart of the different tissues from the pathological wall (i.e., lipid pool, fibrous tissue, calcification), and also to consider their mechanical properties. The applications are described in more detail below. The method and system of the present invention would make it possible to merge geometrical and mechanical data (elasticity and viscosity) in order to study the evolution of pathology and to diagnose it at an early stage of the disease. This predictive approach could show a greater sensitivity than measurement of the intima-media thickness by echography. It would also make it possible to study the effect of medication on the pathology.

The method and system of the present invention may further be used for the study of tumors in vivo (breast, prostate, skin, etc.). The embodiments of the present invention constitute a promising tool for the characterization of the mechanical properties of cancerous breast nodules and prostate tumors since these structures are confined in a mechanically semi-homogeneous medium. The present method and system can provide viscoelasticity measurements with a better defined contrast which would allow an important improvement on the sensitivity, specificity and accuracy of known methods. The present invention can also be used to assess tumor viscoelasticity.

For skin cancers, embodiments of the present invention provide a tool for the geometrical and mechanical characterization of skin tumors by taking advantage of the resonance of a confined structure which depends on the geometry and the mechanical properties of the tumor. The present invention can enable quantification of a tumor's geometry (e.g., diameter, thickness, aspect ratio) and its absolute mechanical properties (e.g., elasticity, viscosity).

The method and system of the present invention may further be used for the study of soft sol-gel materials in a laboratory instrument: The study of the mechanical properties and the rheological behaviour of viscous soft solids remain essential in various applications like quality control, material development and design etc. In the case of blood, the rheological study of coagulation is useful for the comprehension of the formation of venous thromboses and better understanding of the clotting process. Embodiments of the present invention permit this type of characterization with improved accuracy and precision and within a wide frequency range, typically between 0.01 Hz - 10 kHz, thus providing a precise characterization of viscoelastic behaviours.

The principle of viscoelasticity characterization of confined material as developed in the present invention can also be used in a rheometric measurement system. Such as a rheological apparatus, which can be portable, can allow the study of the viscosity and elasticity of soft solid materials or pseudo-solids. The potential applications of such a rheological system cover a large number of fields including the biomedical field for in vitro or ex-vivo characterization of soft biomaterials for diagnosis or research, and for characterizing in vitro blood coagulation and its process (speed of coagulation, viscosity, elasticity etc.) during a blood transfusion for example. The present invention allows monitoring parameters such as the time of coagulation (time between the beginning of coagulation and the time at which the transition between the liquid and solid phase occurs), the time of formation of coagulation (time between the beginning of coagulation and when the values of viscoelasticity reach a stabilization plateau), and the temporal evolution of elasticity and viscosity. Another field of application is the industrial field for the rheological characterization of soft materials and sol-gel transition. Shear-wave induced resonance techniques can be integrated into a rheological characterization apparatus to measure viscoelasticity of various materials used in various industries such as food, chemical, pharmaceutical, material and polymer. This apparatus can serve in research and development laboratories to design and study materials, in control laboratories to monitor the mechanical properties of soft industrial materials and on-line to measure in real time and automatically the consistency of manufactured products. The resulting information can be integrated in an automatic control process to adjust the key manufacturing parameters to satisfy production specifications.

The method and system of the present invention may further be used for therapeutic interventions in vivo, as functions (i.e., adhesion, differentiation, secretion, migration, proliferation, apoptosis, permeability, remodeling, gene expression, etc.) of biological cells are modulated by mechanical forces. Embodiments of the present invention (e.g., repetitive complex shear-wave resonance eigenmodes applied on biological structures) may induce therapeutic mechanotransduction modifying the functions of cells (abnormal cells such as cancer cells, proliferative atherosclerotic cells, etc).

The method and system of the present invention may further be used for promoting the efficiency of clot dissolving drugs in vivo. The present invention (e.g., repetitive complex shear-wave resonance eigenmodes applied on vascular thrombi) may be used to promote the effect of clot dissolving drugs and hence have a therapeutic impact.

It should be appreciated that the invention is not limited to the particular embodiments described and illustrated herein but includes all modifications and variations falling within the scope of the invention as defined in the appended claims.

**Claims**

1. A system for characterizing viscoelastic properties of a first medium which is included within a second medium, the first and second medium having different mechanical properties, the system comprising:

    a shear wave generator (12),
    a detection unit (16),
    a synchronizing unit; and
    a processor (18)
    **characterized** in that
    said shear wave generator (12) comprising a vibrator (24) connected to a contact surface to said second medium and configured to generate incident shear waves at a plurality of frequencies that are applied to the second medium, said shear waves are oriented with respect to the first medium according to a geometry of said first medium and scattered within said first medium selectively inducing mechanical resonances within said first medium,
    said detection unit (16) configured to detect the induced mechanical resonances within said first medium, extract the resonance frequencies of the first medium from the spectrum of displacements of the first medium and generate a shear waves induced resonance frequencies spectrum;
    said synchronizing unit coupled to said generator and said detection unit to sequentially synchronize acquisitions of said induced mechanical resonances and said shear waves generation; and
said processor (18) configured to calculate viscoelastic properties of the first medium using spectral response of said resonance spectrum, a displacement amplitude of said generated incident shear waves, a density of said first medium and second medium, said geometry of said first medium, and mechanical properties of said second medium through an inverse problem using a theoretical model, wherein said model is selected based on said geometry of said first medium.

2. The system of claim 1, wherein said shear wave generator is adapted to generate shear waves in a frequency range comprised between 0.01 and 3500 Hz, preferably between 10 Hz and 2000 Hz, more preferably between 50 Hz and 1000 Hz.

3. The system according to claim 1 or 2, wherein said first medium has a longitudinal axis and said shear wave generator is adapted to generate shear waves substantially parallel to said longitudinal axis or shear waves oriented substantially perpendicularly to said longitudinal axis.

4. The system according to claim 1 or 2, wherein said first medium is substantially spherical and said shear wave generator is adapted to generate torsional shear waves or planar shear waves.

5. The system according to any one of claims 1 to 4, wherein said shear wave generator (12) comprises surface being made to move in such a way as to generate shear waves, or said shear wave generator (12) comprises a rigid plate (22) having a surface contacting the second medium, and a vibrator (24) actuating said plate.

6. The system according to any one of claims 1 to 5, wherein said shear wave generator (12) is adapted to generate non-focalized shear waves or focalized shear waves, said shear waves being harmonic or transient waves.

7. The system according to any one of claims 1 to 6, wherein said first medium has a diameter comprised between 1 mm and 150 mm, preferably between 5 mm and 20 mm.

8. The system according to any one of claims 1 to 7, wherein said first medium has a shear modulus comprised between 10 Pa and 100 000 Pa, preferably between 200 Pa and 20 000 Pa.

9. A method for measuring the viscoelastic properties of a first medium which is included within a second medium, the first and second medium having different mechanical properties, comprising:

generating shear waves using a vibrator connected to a contact surface to said second medium to generate incident shear waves that are applied to the second medium at a plurality of frequencies, said shear waves are oriented with respect to the first medium according to a geometry of said first medium and scattered within said first medium selectively inducing mechanical resonances of said first medium;
detecting the spectrum of displacements within said first medium to extract the resonance frequencies of the first medium and generate a shear wave induced resonance frequency spectrum of displacements;
sequentially synchronizing acquisitions of the mechanical resonances and the shear waves generation; and calculating viscoelastic properties of said first medium using spectral response of said resonance spectrum, a displacement amplitude of said generated shear waves, a density of said first and second medium, geometry of said first medium, and mechanical properties of said second medium through an inverse problem using a theoretical model, wherein said model is selected based on said geometry of said first medium.

10. The method as claimed in claim 9 wherein said first medium is a confined pathology.

11. The method as claimed in claim 9 wherein said second medium is a container.

12. The method as claimed in any one of claims 9 to 11 wherein an elastographic image of the first medium or heterogeneity is obtained from eigenmodes of the displacement spectrum.

13. The method according to any one of claims 9 to 12, wherein said generating shear waves comprises applying to the second medium, or container one of:

i) shear waves substantially parallel to a longitudinal axis of the first medium or heterogeneity;
ii) shear waves oriented substantially perpendicularly to said longitudinal axis;

iii) shear waves substantially circumferential to the first medium or heterogeneity; and
iv) planar shear waves.

14. The method according to any one of claims 9 to 13, wherein said generating shear waves comprises applying shear waves in a frequency range between 0.01 Hz and 3500 Hz, preferably between a few Hertz and 2 kHz, more preferably between 50 Hz and 1000 Hz.

**Patentansprüche**

1. Ein System zur Bestimmung von viskose-elastischen Eigenschaften eines Mediums, welches sich in einem zweiten befindet, beide Medien haben verschiedene mechanische Eigenschaften, das beschriebene System beinhalten

   • einen Transversalwellenerzeuger (12),
   • eine Detektionseinheit (16),
   • eine Synchronisierungseinheit; und
   • einen Prozessor (18)

   beschrieben in dem besagten Transversalwellenerzeuger (12), bestehend aus einem Schwingungserzeuger (24) verbunden mit einer Kontaktoberfläche zu dem beschriebenen zweiten Medium und konfiguriert um Stör-Transversalwellen mit einer Vielzahl von Frequenzen zu generieren, welche auf das zweite Medium angewendet werden, besagte Transversalwellen sind auf das erste Medium bezogen an der Geometrie orientiert, werden im ersten Medium gestreut und erzeugen selektive mechanische Resonanzfrequenzen, beschriebene Detektionseinheit (16) ist so konfiguriert, um die induzierten mechanischen Resonanzen im ersten Medium zu detektieren, die Resonanzfrequenzen aus dem verschobenen Spektrum des ersten Mediums zu extrahieren und ein von den Transversalwellen induziertes Spektrum der Resonanzfrequenzen zu generieren;
   Besagte Synchronisierungseinheit verbinden den Generator und die Detektionseinheit zur sequenziellem synchronisierten Erfassung der induzierten mechanischen Resonanzen und der Transversalwellen;
   Besagter Prozessor (18) ist so konfiguriert um die viskose-elastischen Eigenschaften des ersten Mediums unter Nutzung der Spektralantwort des Resonanzspektrum, der Ablenkungsamplitude der induzierten Transversalwellen., der Dichte des ersten Mediums und des zweiten Mediums, der Geometrie des Ersten Mediums, den mechanischen Eigenschaften des zweiten Mediums durch die Lösung eines inversen Problems durch Nutzung eines theoretischen Problems, wobei das Model auf Grund der Geometrie des ersten Mediums ausgewählt wird.

2. Besagter Transversalwellenerzeuger, wie im System aus Anspruch 1 beschrieben, erzeugt entsprechende Wellen in den Frequenzbereichen 0.01 bis 3500Hz, 10Hz bis 2000Hz und 50Hz bis 1000 Hz.

3. Wie im Anspruch 1 und 2 angegeben, erzeugt der angepasste Transversalwellengenerator in dem ersten Medium, welches eine Ausrichtung in der Längsachse besitzt, Transversalwellen parallel zur Längsachse oder lotrecht zu dieser.

4. Wie im Anspruch 1 und 2 angegeben, erzeugt der angepasste Transversalwellengenerator in dem ersten Medium, welches sphärisch ist, torsions oder planare Transversalwellen.

5. Wie im Anspruch 1 bis 4 angegeben, umfasst der angepasste Transversalwellengenerator (12) eine Oberflache, welche so beschaffen ist, dass sie Transversalwellen erzeugt, oder einer steifen Platte(22), dessen Oberfläche Kontakt zu dem zweiten Medium hat, und einem Schwingungserzeuger (24), welcher die Platte anregt.

6. Wie im Anspruch 1 bis 5 angegeben, erzeugt der angepasste Transversalwellengenerator (12) nicht-fokussierte oder fokussierte, transiente oder harmonische Transversalwellen.

7. Wie im Anspruch 1 bis 6 angegeben, kann das erste Medium Durchmesser von 1mm bis 150mm oder 5mm bis 20mm umfassen.

8. Wie im Anspruch 1 bis 7 angegeben, kann das erste Medium einen Gleitmodul von 10 Pa bis 100.000 Pa oder 200Pa und 20.000 Pa betragen.

9. Eine Methode zur Messung der viskose-elastischen Eigenschaften des ersten Mediums, welches im zweiten Medium beinhalten ist, ist das das erste und zweite Medium folgende verschiedene mechanische Eigenschaften aufweisen:

Transversalwellen erzeugen mittels eines durch eine Kontaktoberfläche an das zweite Medium angeschlossenen Schwingungserzeugers, eine Vielzahl von Frequenzen, besagte Transversalwellen orientieren sich nach der Geometrie des ersten Mediums, und

streuen im ersten Medium um selektierte mechanische Resonanzen im ersten Medium zu induzieren;

Die Detektion des Streuspektrums und das Extrahieren der Resonanzfrequenzen im ersten Medium und Erzeugung von transversalwellen-induzierten Resonanzfrequenzspektrums;

Sequenziell synchronisierte Erfassung der mechanischen Resonanzen und der Transversalwellenerzeugung;

Berechnung der viskose-elastischen Eigenschaften des ersten Mediums unter Nutzung der Spektralantwort des Resonanzspektrums, einer Auslenkungsamplitude der erzeugten Transversalwellen, einer Dichte des ersten und zweiten Mediums, der Geometrie des ersten Mediums, der mechanischen Eigenschaften des zweiten Mediums durch die Lösung eines inversen Problems unter Zuhilfenahme eines theoretischen Models, wobei besagtes Model basierend auf der Geometrie des ersten Mediums bestimmt wird.

10. Wie in Anspruch 9 angegeben, beinhaltet die Methode, dass das erste Medium eine abgeschlossene Symptomatik ist.

11. Wie in Anspruch 9 angegeben, beinhaltet die Methode, dass das zweite Medium ein Behälter ist.

12. Wie in Anspruch 9 bis 11 angegeben, beinhaltet die Methode, dass ein elastrografisches Abbild des ersten Mediums oder die Heterogenität von der Eigenform des Ablenkungsspektrums gewonnen wird.

13. Wie in Anspruch 9 bis 12 angegeben beinhaltete Methode, wobei besagte Erzeugung von Transversalwellen folgende Anwendung auf das zweite medium oder den Behalten umfasst:

I. Transversalwellen im Wesentlichen parallel oder längst zur Achse des ersten Mediums oder heterogen
II. Transversalwellen im Wesentlichen lotrecht orientiert zur Längsachse
III. Transversalwellen im wesentlichen peripher zum ersten Medium oder heterogen
IV. Planare Transversalwellen

14. Wie in Anspruch 9 bis 13 angegeben beinhaltete Methode, wobei besagte erzeugte Transversalwellen die Anwendung der Wellen in den Frequenzbereichen 0,01Hz und 3500Hz, wenigen Hertz bis 2KHz und 50Hz bis 1000Hz umfasst.

## Revendications

1. Un système pour caractériser la viscoélasticité d'un premier milieu inclus dans un second milieu, les premiers et seconds milieux ayant des propriétés mécaniques différentes, et le système comprenant :

un générateur d'ondes de cisaillement (12),
une unité de détection (16),
une unité de synchronisation; et
un processeur (18)

**caractérisé en ce que**
le dit générateur d'ondes de cisaillement (12) comprend un vibreur (24) connecté à une surface de contact audit second milieu et configuré pour générer des ondes de cisaillement incidentes à une pluralité de fréquences qui sont appliquées au second milieu, cesdites ondes de cisaillement sont orientées relativement au premier milieu selon la géométrie de ce premier milieu et sont diffractées dans ledit premier milieu en induisant sélectivement une résonance mécanique dudit premier milieu,
ladite unité de détection est configurée pour détecter les résonances mécaniques induites dans le premier milieu, extraire les fréquences de résonance du premier milieu du spectre de déplacement du premier milieu et générer un spectre de fréquences de résonances induites par des ondes de cisaillement;
ladite unité de synchronisation couplée aux dits générateur et unité de détection synchronise séquentiellement les acquisitions desdites résonances mécaniques induites et le dit générateur d'ondes de cisaillement; et
ledit processeur (18) est configuré pour calculer les propriétés viscoélastiques du premier milieu en utilisant la réponse spectrale dudit premier milieu, l'amplitude de déplacement desdites ondes de cisaillement incidentes générées, les densités desdits premier et second milieux, la géométrie du premier milieu et les propriétés mécaniques du second milieu à travers un problème inverse utilisant un modèle théorique sélectionné en se basant sur la géométrie du dit premier milieu.

2. Le système de la revendication 1, où ledit générateur d'ondes de cisaillement est adapté pour générer des ondes de cisaillement dans une gamme fréquentielle comprise entre 0.01 Hz et 3500 Hz, préférablement entre 10 Hz et 2000 Hz et idéalement entre 50 Hz et 1000 Hz.

3. Le système selon la revendication 1 ou 2, où ledit premier milieu a un axe longitudinal et ledit générateur d'ondes de cisaillement est adapté pour générer des ondes de cisaillement substantiellement parallèles audit axe longitudinal ou des ondes de cisaillement substantiellement perpendiculaires audit axe longitudinal.

4. Le système selon la revendication 1 ou 2, où ledit premier milieu est substantiellement de forme sphérique et ledit générateur d'ondes de cisaillement est adapté pour générer des ondes de cisaillement de torsion ou des ondes de cisaillement planes.

5. Le système selon l'une des revendications 1 à 4, où ledit générateur d'ondes de cisaillement (12) comprend une surface conçue pour se déplacer de telle manière à générer des ondes de cisaillement, ou ledit générateur d'ondes de cisaillement (12) comprend une plaque rigide (22) ayant une surface de contact avec le second milieu, et un vibreur (24) actionnant ladite plaque.

6. Le système selon l'une des revendications 1 à 5, où ledit générateur d'ondes de cisaillement (12) est adapté pour générer des ondes de cisaillement non focalisées ou des ondes de cisaillement focalisés, les dites ondes de cisaillement étant des ondes harmoniques ou transitoires.

7. Le système selon l'une des revendications 1 à 6, où ledit premier milieu a un diamètre compris entre 1 mm et 150 mm, préférablement entre 5 mm et 20 mm.

8. Le système selon l'une des revendications 1 à 7, où ledit premier milieu a un module de cisaillement compris entre 10 Pa et 100 000 Pa, préférablement entre 200 Pa et 20 000 Pa.

9. Une méthode pour mesurer la viscoélasticité d'un premier milieu inclus dans un second milieu, lesdits premier et second milieux ayant des propriétés mécaniques différentes, comprenant :

une génération d'ondes de cisaillement en utilisant un vibreur connecté à travers une surface de contact au second milieu pour générer des ondes de cisaillement incidentes qui sont appliquées au second milieu à une pluralité de fréquences, lesdites ondes de cisaillement étant orientées relativement au premier milieu en fonction de la géométrie dudit premier milieu et diffractées dans ledit premier milieu de façon à induire sélectivement les résonances mécaniques du dit premier milieu;
une détection du spectre de déplacement dans le dit premier milieu pour extraire les fréquences de résonance du premier milieu et générer un spectre des fréquences de résonance des déplacements induites par les ondes de cisaillement;
une synchronisation séquentielle de l'acquisition des résonances mécaniques et de la génération des ondes de cisaillement; et
un calcul des propriétés viscoélastiques dudit premier milieu en utilisant la réponse spectrale dudit spectre de résonance, l'amplitude de déplacement des dites ondes de cisaillement générées, une densité desdits premier et second milieux, une géométrie dudit premier milieu et les propriétés mécaniques dudit second milieu à travers un problème inverse utilisant un modèle théorique, où ledit modèle est sélectionné en se basant sur ladite géométrie du premier milieu.

10. La méthode de la revendication 9, où ledit premier milieu est une pathologie confinée.

11. La méthode de la revendication 9, où ledit second milieu est un contenant.

12. La méthode selon l'une des revendications 9 à 11, où une image élastographique du premier milieu ou d'une hétérogénéité est obtenue à partir des modes propres du spectre de déplacement.

**13.** La méthode selon l'une des revendications 9 à 12, o<u ladite génération d'ondes de cisaillement comprend l'application au second milieu ou au contenant :

   i) d'ondes de cisaillement substantiellement parallèles à un axe longitudinal du premier milieu ou de l'hétérogénéité;
   ii) d'ondes de cisaillement substantiellement perpendiculaires à cedit axe longitudinal;
   iii) d'ondes de cisaillement substantiellement circonférentielles au premier milieu ou à l'hétérogénéité; ou
   iv) d'ondes de cisaillement planes.

**14.** La méthode selon l'une des revendications 9 à 13, où ladite génération d'ondes de cisaillement comprend l'application d'ondes de cisaillement dans une gamme fréquentielle comprise entre 0.01 Hz et 3500 Hz, préférablement entre quelques Hertz et 2 kHz, idéalement entre 50 Hz et 1000 Hz.

Fig-1

Fig-2

EP 2 310 829 B1

*Fig-3*

Fig-4a

Fig-4b

*12*

$S_2$  $P_3$  $P_2$  $P_1$

$B_1$

*24*

*36*  $A$

*38*

$S_1$

*34*

*22*  $B$

——— vibration

▨▨▨▨ manual positioning

*Fig-4c*

Fig. 5a

Fig. 5b

Fig. 5c

Fig. 6

*Fig. 7a*

*Fig. 7b*

*Fig. 8a*

*Fig. 8b*

12

15

14

(y)

16

*Fig. 8c*

Suspected nodule
(11.0 mm)

HIGH

Resonance Factor

B-mode image

Superposition of B-mode image with
the stationary displacement image
At resonance

*Fig. 8d*

*100*

*102*

Inducing vibrations into a heterogeneity within a sample

*104*

Extracting the eigenfrequencies of the heterogeneity from the displacement spectra

*106*

Imaging the shape of the eigenmodes of the heterogeneity corresponding to the eigenfrequencies

Fig-9

PC  *42*  USB

Communication port  *40*  *12*

Processor  Amplifier  Shear Wave generation system

*44*  Acquisition card  N/A

Input/output  N/A  Measured signal conditioning  Sample or body

*46*  Ultrasound, Laser, Microphone, Magnetic resonance imaging,  *48*  *14*

*50*

Monitoring

Fig-10

Fig-11a

Fig-11b

Fig-12

Fig-13a

Fig-13b

1st vibration eigenmode corresponding to f1 = 100.0 Hz

2nd vibration eigenmode corresponding to f2 = 158.0 Hz

3rd vibration eigenmode corresponding to f3 = 230.0 Hz

Fig_14

Fig_15

3D REPRESENTATION OF THE EIGENMODES — 1D MESURED STATIONARY DISPLACEMENT PROFILES

1st vibration eigenmode corresponding to f1 = 100.0 Hz

2nd vibration eigenmode corresponding to f2 = 158.0 Hz

3rd vibration eigenmode corresponding to f3 = 230.0 Hz

FIG_16

1st resonance frequency vs. elasticity

_Fig-17a_

Q Factor (quality factor) vs. viscosity

_Fig-17b_

EP 2 310 829 B1

**Sample preparation**
Include the material sample into a
conformed cylindrical shape contained into a soft material

**Multi-frequency excitation**
Induce harmonic or transient incident shear waves
at different frequencies with known amplitudes

**Measurement**
Measuring, for each generated shear wave, the
sample out of plane displacement, velocity acceleration or
pressure ( depending on the used measurement technology )

**Spectral post-processing**
Process the measured data to extract the sample
spectral response : resonance frequencies, quality factor...

**Viscoelastic material characterization**
Solve an inverse problem involving the measured
data and a theoretical model to assess both elasticity and viscosity
on a large frequency range

yes     Is the surrounding     no
medium know ?

**Characterization of the
sample material**
Viscoelastic properties
and rheological law

**Characterization of both sample
and surrounding materials**
Viscoelastic properties
and rheological laws

*Fig_17c*

Fig-18a

Fig. 18b

Fig_18c

Fig_18d

Fig-18e

Fig-19a

Fig-19b

Fig-19c

Fig-19d

Fig-19ℓ

Spectrum of displacement for 0° incidence

Fig. 20a

Displacement Field (mm) at $f_0$ = 61 Hz

_Fig-20b_

Displacement Field (mm) at f = 86 Hz

_Fig-20c_

Incident torsional wave

_Fig-21_

1st resonance mode at 130 Hz

Fig. 22a

2nd resonance mode at 169 Hz

Fig. 22b

Fig. 23a

Fig. 23b

Fig. 23c

Fig. 23d

*Fig- 24a*

*Fig- 24b*

*Fig- 24c*

Fig-25a

Fig-25b

Fig. 25c

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2005004463 A
- US 5099848 A
- JP 2000187024 A
- US 5592085 A
- US 6037774 A

### Non-patent literature cited in the description

- **A. SAMANI et al.** *Phys. Med. Biol.,* 2007, vol. 52, 1565-1576
- **J. M. RUBIN et al.** *Ultrasound Med. Biol.,* 2006, vol. 25 (9), 1179-1186
- **K. J. PARKER et al.** *Ultrasound Med. Biol.,* 1990, vol. 16, 241-246
- **R. MUTHUPILLAL et al.** *Science,* 1995, vol. 269 (5232), 1854-1857
- **K. NIGHTINGALE et al.** *Ultrasound Med. Biol.,* 2002, vol. 28, 227-235
- **J. BERCOFF et al.** *Appl. Phys. Lett.,* 2004, vol. 84, 2202-2204
- **THE ARTICLE OF S. CHEN et al.** Quantifying elasticity and viscosity from measurement of shear wave speed dispersion. *journal of the acoustical society of America,* 2004, vol. 115 (6), 2781-5
- **K. J. PARKER et al.** A unified view of imaging the elastic properties of tissue. *The journal of the acoustical society of America,* 2005, vol. 117 (5), 2705-12
- **C. SCHMITT et al.** Characterization of Time-Varying Mechanical Viscoelastic Parameters of Mimicking Deep Vein Thrombi with 2D Dynamic Elastography. *Ultrasonics Symposium,* 2007, 1009-12
- **S. J. KIRKPATRICK et al.** Imaging the mechanical stiffness of skin lesions by in vivo acousto-optical elastography. *Optics Express,* 2006, vol. 14 (21), 9770-9779
- **S.J. KIRKPATRICK ; R.K. WANG.** Imaging the mechanical stiffness of skin lesions by in vivo acousto-optical elastography. *Optics Express,* 2006, vol. 14 (21), 9770-9779
- **S. CATHELINE et al.** Measuring of viscoelastic properties of homogeneous soft solid using transient elastography: an inverse problem approach. *J. Acoust. Soc. Am.,* 2004, vol. 116 (6), 3734-3741
- **J.D. ACHENBACH.** *Wave Propagation in Elastic Solids,* 1973
- **P.M. MORSE ; H. FESHBACH.** Methods of Theoretical Physics. McGraw-Hill, 1953, 1376
- **A.R. HADJ HENNI ; C. BACON.** In-plane vibration of thin elliptic plates submitted to uniform pulsed microwave irradiations. *Journal of Sound and Vibration,* 2007, vol. 299, 298-313
- **J.D. ACHENBACH.** Wave Propagation in Elastic Solids. 1973, 55
- **L. KNOPOFF.** Scattering of shear waves by spherical obstacles. *Geophysics,* 1959, vol. 24, 209-219
- **F. SCHWAB.** Scattering of shear waves by small transeismic obstacles. *Geophysics,* 1965, vol. 30, 24-31